# EUROPEAN PATENT APPLICATION

(11) **EP 2 272 511 A1**
(43) Date of publication of application: **12.01.2011**
(21) Application number: 10174260.9
(22) Date of filing: 08.05.2007
(51) Int. Cl.: A61K 31/4196, A61K 31/513, A61K 31/40, A61K 31/282, A61K 31/517, A61K 31/4985, A61P 35/00

(54) **Combination comprising an iron chelator and an anti-neoplastic agent and use thereof**

(30) Priority: 09.05.2006 US 746788 P
(62) Divisional of application: 07728908.0
(71) Applicant: Novartis AG, 4056 Basel (CH); Novartis Pharma GmbH, 1230 Wien (AT)
(72) Inventor: Nick, Hanspeter, 4202, Duggigen (CH)
(74) Representative: Roth, Peter Richard

(57) **Abstract**

The present invention relates to a combination comprising a a substituted 3,5-diphenyl-1,2,4-triazole, e.g. 4-[3,5-bis(2-hydroxyphenyl)-[1,2,4]triazol-1-yl]benzoic acid and at least one anti-neoplastic agent and to the use of said combination for the preparation of a medicament for the treatment of proliferative diseases.

## Description

The invention relates to a method of preventing or treating diseases that are associated with the growth and/or proliferation of neoplastic cells and/or tissue and/or triggered by persistent angiogenesis in a mammal, particularly a human, with a combination of pharmaceutical agents which comprises:
(a) an iron chelator; and
(b) one or more pharmaceutically active agents.

The invention relates to a combination of pharmaceutical agents which comprises:
(a) an iron chelator; and
(b) one or more pharmaceutically active agents.

In one embodiment, the invention relates to the use of a combination of pharmaceutical agents which comprises:
(a) an iron chelator; and
(b) one or more pharmaceutically active agents,
   for the manufacture of a medicament for use in the treatment of a proliferative disease.

The invention further relates to pharmaceutical compositions comprising:
(a) an iron chelator;
(b) a pharmaceutically active agent; and
(c) a pharmaceutically acceptable carrier.

The present invention further relates to a commercial package or product comprising:
(a) a pharmaceutical formulation of an iron chelator; and
(b) a pharmaceutical formulation of a pharmaceutically active agent for simultaneous, concurrent, separate or sequential use.

The combination partners (a) and (b) can be administered together, one after the other or separately in one combined unit dosage form or in two separate unit dosage forms. The unit dosage form may also be a fixed combination.

Chemotherapy for the treatment of proliferative diseases is known in the art.

Surprisingly, it has been found that the antineoplastic effect, in particular in the treatment of a proliferative disease, especially a liquid, e.g. multiple myeloma, myelodisplactic syndrome or a solid tumor disease, e.g. liver cancer such as for example, hepatocellular carcinoma, e.g. which is refractory to other chemotherapeutics known as antineoplastic agents, of a combination of an anti-neoplastic agent with an iron chelator, e.g. a substituted 3,5-diphenyl-1,2,4-triazole alone, e.g. 4-[3,5-bis(2-hydroxyphenyl)-[1,2,4]triazol-1-yl]benzoic acid is greater than the effect of a therapy using either the anti-neoplastic agent or the iron chelator, e.g. a substituted 3,5-diphenyl-1,2,4-triazole alone, e.g. 4-[3,5-bis(2-hydroxyphenyl)-[1,2,4]triazol-1-yl]benzoic acid, alone.

### The Combinations

It will be understood that references to the components (a) and (b) are meant to also include the pharmaceutically acceptable salts of any of the active substances. If active substances comprised by components (a) and/or (b) have, for example, at least one basic center, they can form acid addition salts. Corresponding acid addition salts can also be formed having, if desired, an additionally present basic center. Active substances having an acid group, e.g., COOH, can form salts with bases. The active substances comprised in components (a) and/or (b) or a pharmaceutically acceptable salts thereof may also be used in form of a hydrate or include other solvents used for crystallization. substituted 3,5-diphenyl-1,2,4-triazoles are the most preferred combination partner (a).

The present invention relates to a combination of pharmaceutical agents which comprises :

### (a) an iron chelator such as a Compound of formula II

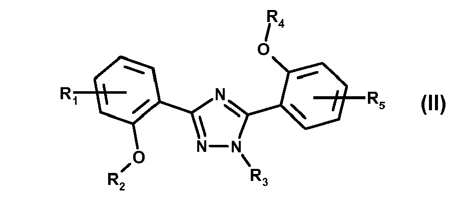

in which
R₁ and R₅ simultaneously or independently of one another are hydrogen, halogen, lower-alkyl, halo-lower alkyl, lower alkoxy, halo-lower alkoxy, carboxyl, carbamoyl, N-lower alkylcarbamoyl, N,N-di-lower alkylcarbamoyl or nitrile; R₂ and R₄ simultaneously or independently of one another are hydrogen, unsubstituted or substituted lower alkanoyl or aroyl, or a radical which can be removed under physiological conditions; R₃ is R₆R₇N-C(O)-lower alkyl, unsubstituted or substituted aryl, aryl-lower alkyl, substituted by N-lower alkylamino, N,N-di-lower alkylamino or pyrrolidino, or unsubstituted or substituted heteroaryl or heteroaralkyl, with the proviso that R₃ is not phenyl or phenyl substituted by halogen, nitro, nitrile, hydroxyl, lower alkyl, halo-lower alkyl, lower alkoxy or lower alkoxycarbonyl if R₂ and R₄ are hydrogen, and R₁ and R₅ are hydrogen, halogen, lower alkyl, halo-lower alkyl, lower alkoxy or nitrile; R₆ and R₇ simultaneously or independently of one another are hydrogen, lower alkyl, hydroxy-lower alkyl, alkoxy-lower alkyl, hydroxyalkoxy-lower alkyl, amino-lower alkyl, N-lower alkylamino-lower alkyl, N,N-di-lower alkylamino-lower alkyl, N-(hydroxy-lower alkyl)amino-lower alkyl, N,N-di(hydroxy-lower alkyl)amino-lower alkyl or, together with the nitrogen atom to which they are bonded, form an azaalicyclic ring and salts thereof; and

### (b) one or more anti-neoplastic agents.

Halogen is, for example, chlorine, bromine or fluorine, but can also be iodine.

The prefix "lower" designates a radical having not more than 7 and in particular not more than 4 carbon atoms.

Alkyl is straight-chain or branched. Per se, for example lower alkyl, or as a constituent of other groups, for example lower alkoxy, lower alkylamine, lower alkanoyl, lower alkylaminocarbonyl, it can be unsubstituted or substituted, for example by halogen, hydroxyl, lower alkoxy, trifluoromethyl, cyclo-lower alkyl, azaalicyclyl or phenyl, it is preferably unsubstituted or substituted by hydroxyl.

Lower alkyl is, for example, *n*-propyl, isopropyl, *n*-butyl, isobutyl, *sec*-butyl, *tert-butyl, n*-pentyl, neopentyl, *n*-hexyl or *n*-heptyl, preferably methyl, ethyl and *n*-propyl. Halo-lower alkyl is lower alkyl substituted by halogen, preferably chlorine or fluorine, in particular by up to three chlorine or fluorine atoms.

Lower alkoxy is, for example, *n*-propoxy, isopropoxy, *n*-butoxy, isobutoxy, *sec*-butoxy, *tert-*butoxy, *n*-amyloxy, isoamyloxy, preferably methoxy and ethoxy. Halo-lower alkoxy is lower alkoxy substituted by halogen, preferably chlorine or fluorine, in particular by up to three chlorine or fluorine atoms.

Carbamoyl is the radical H₂N-C(O)-, N-lower alkylcarbamoyl is lower alkyl-HN-C(O)- and N,N-di-lower alkylcarbamoyl is di-lower alkyl-N-C(O)-.

Lower alkanoyl is HC(O)- and lower alkyl-C(O)- and is, for example, acetyl, propanoyl, butanoyl or pivaloyl.

Lower alkoxycarbonyl designates the radical lower alkyl-O-C(O)- and is, for example, *n*-propoxycarbonyl, isopropoxycarbonyl, *n*-butoxycarbonyl, isobutoxycarbonyl, *sec*-butoxycarbonyl, *tert*-butoxycarbonyl, *n*-amyloxycarbonyl, isoamyloxycarbonyl, preferably methoxycarbonyl and ethoxycarbonyl.

Aryl, per se, for example aryl, or as a constituent of other groups, for example aryl-lower alkyl or aroyl, is, for example, phenyl or naphthyl, which is substituted or unsubstituted. Aryl is preferably phenyl which is unsubstituted or substituted by one or more, in particular one or two, substituents, for example lower alkyl, lower alkoxy, hydroxyl, nitro, amino, halogen, trifluoromethyl, carboxyl, lower alkoxycarbonyl, amino, N-lower alkylamino, N,N-di-lower alkylamino, aminocarbonyl, lower alkylaminocarbonyl, di-lower alkylaminocarbonyl, heterocycloalkyl, heteroaryl or cyano. Primarily, aryl is unsubstituted phenyl or naphthyl, or phenyl which is substituted by lower alkyl, lower alkoxy, hydroxyl, halogen, carboxyl, lower alkoxycarbonyl, N,N-di-lower alkylamino or heterocycloalkylcarbonyl.

Aroyl is the radical aryl-C(O)- and is, for example, benzoyl, toluoyl, naphthoyl or p-methoxybenzoyl.

Aryl-lower alkyl is, for example, benzyl, p-chlorobenzyl, o-fluorobenzyl, phenylethyl, p-tolylmethyl, p-dimethylaminobenzyl, p-diethylaminobenzyl, p-cyanobenzyl, p-pyrrolidinobenzyl.

Heterocycloalkyl designates a cycloalkyl radical having 3 to 8, in particular having from 5 to not more than 7, ring atoms, of which at least one is a heteroatom; oxygen, nitrogen and sulfur are preferred. Azaalicyclyl is a saturated cycloalkyl radical having 3-8, in particular 5-7, ring atoms, in which at least one of the ring atoms is a nitrogen atom. Azaalicyclyl can also contain further ring heteroatoms, e.g. oxygen, nitrogen or sulfur; it is, for example, piperidinyl, piperazinyl, morpholinyl or pyrrolidinyl. Azaalicyclyl radicals can be unsubstituted or substituted by halogen or lower alkyl. The azaalicyclyl radicals bonded via a ring nitrogen atom, which are preferred, are, as is known, designated as piperidino, piperazino, morpholino, pyrrolidino etc.

Heteroaryl per se, for example heteroaryl, or as a constituent of other substituents, for example heteroaryl-lower alkyl, is an aromatic radical having from 3 to not more than 7, in particular from 5 to not more than 7, ring atoms, in which at least one of the ring atoms is a heteroatom, e.g. pyrrolyl, imidazolyl, triazolyl, tetrazolyl, oxazolyl, thiazolyl, furanyl, thiophenyl, pyridyl, pyrazinyl, oxazinyl, thiazinyl, pyranyl or pyrimidinyl. Heteroaryl can be substituted or unsubstituted. Heteroaryl which is unsubstituted or substituted by one or more, in particular one or two, substituents, for example lower alkyl, halogen, trifluoromethyl, carboxyl or lower alkoxycarbonyl, is preferred.

Heteroaryl-lower alkyl designates a lower alkyl radical in which at least one of the hydrogen atoms, preferably on the terminal C atom, is replaced by a heteroaryl group if the alkyl chain contains two or more carbon atoms.

N-lower alkylamino is, for example, *n*-propylamino, *n*-butylamino, *i*-propylamino, i-butylamino, hydroxyethylamino, preferably methylamino and ethylamino. In N,N-di-lower alkylamino, the alkyl substituents can be identical or different. Thus N,N-di-lower alkylamino is, for example, N,N-dimethylamino, N,N-diethylamino, N,N-methylethylamino, N-methyl-N-morpholinoethylamino, N-methyl-N-hydroxyethylamino, N-methyl-N-benzylamino.

Salts of compounds of the formula I are, in particular, pharmaceutically acceptable salts, especially salts with bases, such as appropriate alkali metal or alkaline earth metal salts, e.g. sodium, potassium or magnesium salts, pharmaceutically acceptable transition metal salts such as zinc salts, or salts with organic amines, such as cyclic amines, such as mono-, di- or tri-lower alkylamines, such as hydroxy-lower alkylamines, e.g. mono-, di- or trihydroxy-lower alkylamines, hydroxy-lower alkyl-lower alkylamines or polyhydroxy-lower alkylamines. Cyclic amines are, for example, morpholine, thiomorpholine, piperidine or pyrrolidine. Suitable mono-lower alkylamines are, for example, ethyl- and *tert*-butylamine; di-lower alkylamines are, for example, diethyl- and diisopropylamine; and tri-lower alkylamines are, for example, trimethyl- and triethylamine. Appropriate hydroxy-lower alkylamines are, for example, mono-, di- and triethanolamine; hydroxy-lower alkyl-lower alkylamines are, for example, N,N-dimethylamino- and N,N-diethylaminoethanol; a suitable polyhydroxy-lower alkylamine is, for example, glucosamine. In other cases it is also possible to form acid addition salts, for example with strong inorganic acids, such as mineral acids, e.g. sulfuric acid, a phosphoric acid or a hydrohalic acid, with strong organic carboxylic acids, such as lower alkanecarboxylic acids, e.g. acetic acid, such as saturated or unsaturated dicarboxylic acids, e.g. malonic, maleic or fumaric acid, or such as hydroxycarboxylic acids, e.g. tartaric or citric acid, or with sulfonic acids, such as lower alkane- or substituted or unsubstituted benzenesulfonic acids, e.g. methane- or p-toluenesulfonic acid. Compounds of the formula I having an acidic group, e.g. carboxyl, and a basic group, e.g. amino, can also be present in the form of internal salts, i.e. in zwitterionic form, or a part of the molecule can be present as an internal salt, and another part as a normal salt.

The compound of formula I wherein R₂ and R₄ are each hydrogen and R₁ and R₅ are hydroxyl and R₃ is phenyl substituted by carboxyl is 4-[3,5-bis(2-hydroxyphenyl)-[1,2,4]triazol-1-yl]benzoic acid, known under the Trademark EXJADE®.

The compounds of formula I and 4-[3,5-bis(2-hydroxyphenyl)-[1,2,4]triazol-1-yl]benzoic acid and pharmaceutically acceptable salts thereof are described in WO97/49395 published on December 31, 1997 and in the US granted patent 6,465,504.

4-[3,5-bis(2-hydroxyphenyl)-[1,2,4]triazol-1-yl]benzoic acid in the free acid form, salts thereof and its crystalline forms are disclosed in the International Patent Publication WO 97/49395, which is hereby incorporated by reference.

The term "anti-neoplastic agents" as used herein includes, but is not limited to an aromatase inhibitor;
i. an anti-estrogen, an anti-androgen or a gonadorelin agonist;
ii. a topoisomerase I inhibitor or a topoisomerase II inhibitor;
iii. a microtubule active agent, an alkylating agent, an anti-neoplastic anti-metabolite or a platin compound;
iv. a compound targeting/decreasing a protein or lipid kinase activity or a protein or lipid phosphatase activity, a further anti-angiogenic compound or a compound which induces cell differentiation processes;
v. monoclonal antibodies;
vi. a cyclooxygenase inhibitor, a bisphosphonate, a heparanase inhibitor, a biological response modifier;
vii. an inhibitor of Ras oncogenic isoforms;
viii. a telomerase inhibitor;
ix. a protease inhibitor, a matrix metalloproteinase inhibitor, a methionine aminopeptidase inhibitor, or a proteasome inhibitor;
x. agents used in the treatment of hematologic malignancies or compounds which target, decrease or inhibit the activity of Flt-3;
xi. an HSP90 inhibitor;
xii. anti-proliferative antibodies;
xiii. a histone deacetylase (HDAC) inhibitor;
xiv. a compound which targets, decreases or inhibits the activity/function of serine/threonine mTOR kinase;
xv. a somatostatin receptor antagonist;
xvi. an anti-leukemic compound;
xvii. tumor cell damaging approaches;
xviii. an EDG binder;
xix. a ribonucleotide reductase inhibitor;
xx. an S-adenosylmethionine decarboxylase inhibitor;
xxi. a monoclonal antibody of VEGF or VEGFR;
xxii. photodynamic therapy;
xxiii. an angiostatic steroid;
xxiv. an implant containing corticosteroids;
xxv. an AT1 receptor antagonist; and
xxvi. an ACE inhibitor.

The term "aromatase inhibitors" as used herein relates to compounds which inhibit the estrogen production, i.e. the conversion of the substrates androstenedione and testosterone to estrone and estradiol, respectively. The term includes, but is not limited to steroids, especially atamestane, exemestane and formestane and, in particular, non-steroids, especially aminoglutethimide, roglethimide, pyridogluthethimide, trilostane, testolactone, ketokonazole, vorozole, fadrozole, anastrozole and, very especially, letrozole. Exemestane can be administered, e.g. in the form as it is marketed, e.g. under the trademark AROMASIN™. Formestane can be administered, e.g. in the form as it is marketed, e.g. under the trademark LENTARON™. Fadrozole can be administered, e.g. in the form as it is marketed, e.g. under the trademark AFEMA™. Anastrozole can be administered, e.g. in the form as it is marketed, e.g. under the trademark ARIMIDEX™. Letrozole can be administered, e.g. in the form as it is marketed, e.g. under the trademark FEMARA™ or FEMAR™. Aminoglutethimide can be administered, e.g. in the form as it is marketed, e.g. under the trademark ORIMETEN™.

The term "anti-estrogens" as used herein relates to compounds which antagonize the effect of estrogens at the estrogen receptor level. The term includes, but is not limited to tamoxifen, fulvestrant, raloxifene and raloxifene hydrochloride. Tamoxifen can be administered, e.g. in the form as it is marketed, e.g. under the trademark NOLVADEX™. Raloxifene hydrochloride can be administered, e.g. in the form as it is marketed, e.g. under the trademark EVISTA™. Fulvestrant can be formulated as disclosed in US 4,659,516 or it can be administered, e.g. in the form as it is marketed, e.g. under the trademark FASLODEX™. A combination of the invention comprising an anti-neoplastic agent which is an anti-estrogen is particularly useful for the treatment of hormone receptor positive breast tumors.

The term "anti-androgens" as used herein, relates to a y substance which is capable of inhibiting the biological effects of androgenic hormones and includes, but is not limited to bicalutamide (CASODEXT™, which can be formulated, e.g. as disclosed in US 4,636,505.

The term "gonadorelin agonist" as used herein includes, but is not limited to abarelix, goserelin and goserelin acetate. Goserelin is disclosed in US 4,100,274 and can be administered, e.g. in the form as it is marketed, e.g. under the trademark ZOLADEX™. Abarelix can be formulated, e.g. as disclosed in US 5,843,901.

The term "topoisomerase I inhibitors" as used herein includes, but is not limited to topotecan, gimatecan, irinotecan, camptothecin, e.g. 9-nitrocamptothecin and the macromolecular camptothecin conjugate PNU-166148, i.e. compound A1 in WO99/17804. Irinotecan can be administered, e.g. in the form as it is marketed, e.g. under the trademark CAMPTOSAR™. Topotecan can be administered, e.g. in the form as it is marketed, e.g. under the trademark HYCAMTIN™_{.}

The term "topoisomerase II inhibitors" as used herein includes, but is not limited to the anthracyclines doxorubicin, including liposomal formulation, e.g. CAELYX™, daunorubicin, including liposomal formulation, e.g. DAUNOSOME™, epirubicin, idarubicin, mytomycin C, pirarubicin, and nemorubicin, the anthraquinones mitoxantrone and losoxantrone, and the podophillotoxines etoposide and teniposide. Etoposide can be administered, e.g. in the form as it is marketed, e.g. under the trademark ETOPOPHOS™. Teniposide can be administered, e.g. in the form as it is marketed, e.g. under the trademark VM 26-BRISTOL™. Doxorubicin can be administered, e.g. in the form as it is marketed, e.g. under the trademark ADRIBLASTIN™. Epirubicin can be administered, e.g. in the form as it is marketed, e.g. under the trademark FARMORUBICIN™. Idarubicin can be administered, e.g. in the form as it is marketed, e.g. under the trademark ZAVEDOS™. Mitoxantrone can be administered, e.g. in the form as it is marketed, e.g. under the trademark NOVANTRON™.

The term "microtubule active agent" relates to microtubule stabilizing agent , microtubule destabilizing agents and microtubule polymerization inhibitors including but not limited to, taxanes, e.g. paclitaxel and docetaxel, the vinca alkaloids, e.g. vinblastine, especially vinblastine sulfate, vincristine, especially vincristine sulfate, and vinorelbine and discodermolide, colchicines and epothilones and derivatives thereof, e.g. epothilone B or derivatives thereof. Paclitaxel can be administered, e.g. in its marketed form, e.g. under the trademark TAXOL™, Docetaxel as TAXOTERE™. Vinblastine sulfate can be administered, e.g. in the form as it is marketed, e.g. under the trademark VINBLASTIN R.P.™. Vincristine sulfate can be administered, e.g. in the form as it is marketed, e.g. under the trademark FARMISTIN™. Also included are the generic forms of paclitaxel as well as various dosage forms of paclitaxel. Generic forms of paclitaxel include, but are not limited to, betaxolol hydrochloride. Various dosage forms of paclitaxel include, but are not limited to albumin nanoparticle paclitaxel marketed as ABRAXANE™, ONXOL™, CYTOTAX™. Discodermolide can be obtained, e.g. as disclosed in U.S. patents US 4,939,168 and US 5,618,487 to Harbor Branch Oceanographic Institute or by chemical synthesis as described, for example, in GB 2280677, WO 98/24429 and U.S. patents US 5,789,605 and US 6,031,133, which are here incorporated by reference. Also included are Epothilone derivatives which are disclosed in U.S. Patent No. 6,194,181, WO 98/10121, WO 98/25929, WO 98/08849, WO 99/43653, WO 98/22461 and WO 00/31247. Especially preferred are Epotholine A and/or B.

The term "alkylating agent", as used herein, includes, but is not limited to, cyclophosphamide, ifosfamide, melphalan or nitrosourea (BCNU or Gliadel), or temozolamide (TEMODAR). Cyclophosphamide can be administered, e.g. in the form as it is marketed, e.g. under the trademark CYCLOSTIN; and ifosfamide as HOLOXAN.

The term "anti-neoplastic anti-metabolite" includes, but is not limited to, 5-fluorouracil (5-FU); capecitabine; gemcitabine; DNA de-methylating agents, such as 5-azacytidine and decitabine; methotrexate; edatrexate; and folic acid antagonists such as, but not limited to, pemetrexed and leucovorin; and thymidilate synthase inhibitors, e.g. raltitrexed (TOMUDEX). Capecitabine can be administered, e.g. in the form as it is marketed, e.g. under the trademark XELODA; and gemcitabine as GEMZAR.

The term "platinum compound" as used herein means carboplatin, cisplatin, strataplatin, oxaliplatin or platinum agents such as ZD0473. Preferably, the platinum compound is cisplatin.

The term "carboplatin" as used herein relates to the antineoplastic agent cis-diamine (1,1-cyclobutane dicarboxylato) platinum(II), which is disclosed, e.g. in US 4,140,707 or by R.C. Harrison et al. in Inorg. Chim. Acta 46, L15 (1980). This drug can be administered, e.g. in the form as it is marketed, e.g. under the trademark CARBOPLAT™ or PARAPLATIN™.

The term "oxaliplatin" as used herein relates to the antineoplastic agent also known as oxalatoplatinum, which is disclosed, e.g. in US 5,716,988. This drug can be administered e.g. in the form as described in the cited US patent or in the form it is marketed, e.g. under the trademark ELOXANTINE™ or 1-OHP™.

The term "cisplatin" as used herein relates to the antineoplastic agent also known as *cis-*diaminedichloroplatinum, which compound and its use as antineoplastic agent is disclosed, e.g. in DE 2,318,020.

The term "compounds targeting/decreasing a protein or lipid kinase activity; or a protein or lipid phosphatase activity; or further anti-angiogenic compounds", as used herein, includes, but is not limited to, protein tyrosine kinase and/or serine and/or theroine kinase inhibitors or lipid kinase inhibitors, for example:
i) compounds targeting, decreasing or inhibiting the activity of the vascular endothelial growth factor-receptors (VEGF), such as compounds which target, decrese or inhibit the activity of VEGF, especially compounds which inhibit the VEGF receptor, such as, but not limited to, 7H-pyrrolo[2,3-d]pyrimidine derivatives, e.g. 7H-pyrrolo[2,3-d]pyrimidine derivative, {6-[4-(4-ethyl-piperazin-1-ylmethyl)-phenyl]-7H-pyrrolo[2,3-d]pyrimidin-4-yl]-((R)-1-phenylethyl)-amine; BAY 43-9006; isolcholine compounds disclosed in WO 00/09495 such as (4-tert-butyl-phenyl)-94-pyridin-4-ylmethyl-isoquinolin-1-yl)-amine; and
ii) compounds targeting, decreasing or inhibiting the activity of the platelet-derived growth factor-receptors (PDGFR), such as compounds which target, decrease or inhibit the activity of PDGFR, especially compounds which inhibit the PDGF receptor, e.g., a N-phenyl-2-pyrimidine-amine derivative, e.g., imatinib, SU101, SU6668 and GFB-111;
iii) compounds targeting, decreasing or inhibiting the activity of the fibroblast growth factor-receptors (FGFR);
iv) compounds targeting, decreasing or inhibiting the activity of the insulin-like growth factor receptor 1 (IGF-1R), such as compounds which target, decrease or inhibit the activity of IGF-IR, especially compounds which inhibit the IGF-1R receptor. Compounds include but are not limited to the compounds disclosed in WO 02/092599 and derivatives thereof of 4-amino-5-phenyl-7-cyclobutyl-pyrrolo[2,3-d]pyrimidine derivatives;
v) compounds targeting, decreasing or inhibiting the activity of the Trk receptor tyrosine kinase family;
vi) compounds targeting, decreasing or inhibiting the activity of the Axl receptor tyrosine kinase family;
vii) compounds targeting, decreasing or inhibiting the activity of the c-Met receptor;
viii) compounds targeting, decreasing or inhibiting the activity of the Ret receptor tyrosine kinase;
ix) compounds targeting, decreasing or inhibiting the activity of the Kit/SCFR receptor tyrosine kinase;
x) compounds targeting, decreasing or inhibiting the activity of the C-kit receptor tyrosine kinases (part of the PDGFR family), such as compounds which target, decrease or inhibit the activity of the c-Kit receptor tyrosine kinase family, especially compounds which inhibit the c-Kit receptor, e.g., imatinib;
xi) compounds targeting, decreasing or inhibiting the activity of members of the c-Abl family and their gene-fusion products, e.g., BCR-AbI kinase, such as compounds which target decrease or inhibit the activity of c-AbI family members and their gene fusion products, e.g., a N-phenyl-2-pyrimidine-amine derivative, e.g. imatinib of formula (N-{5-[4-(4-methyl-piperazino-methyl)-benzoylamido]-2-methylphenyl}-4-(3-pyridyl)-2-pyrimidine-amine, PD180970, AG957, NSC 680410 or PD173955 from ParkeDavis; nilotinib or 4-methyl-N-[3-(4-methylimidazol-1-yl)-5-(trifluoromethyl)phenyl]-3-[(4-pyridin-3-ylpyrimidin-2-yl)amino] benzamide, dasatinib (BMS354825) or *N*-(2-chloro-6-methylphenyl)-2-[[6-[4-(2-hydroxyethyl)-1-piperazinyl]-2-methyl-4-pyrimidinyl]amino]-5-thiazolecarboxamide monohydrate, bosutinib of the following formula 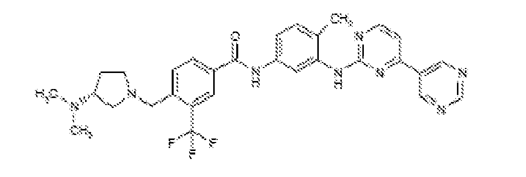 and INNO-406 of the following formula 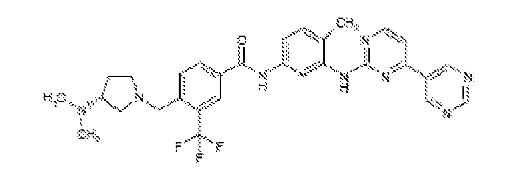
xii) compounds targeting, decreasing or inhibiting the activity of members of the protein kinase C (PKC) and Raf family of serine/threonine kinases, members of the MEK, SRC, JAK, FAK, PDK and Ras/MAPK family members, or PI(3) kinase family, or of the PI(3)-kinase-related kinase family, and/or members of the cyclin-dependent kinase family (CDK) and are especially those staurosporine derivatives disclosed in U.S. Patent No. 5,093,330, e.g., midostaurin; examples of further compounds include, e.g., UCN-01; safingol; BAY 43-9006; Bryostatin 1; Perifosine; Ilmofosine; RO 318220 and RO 320432; GO 6976; Isis 3521; LY333531/LY379196; isochinoline compounds, such as those disclosed in WO 00/09495; FTIs; PD184352 or QAN697, a P13K inhibitor;
xiii) compounds targeting, decreasing or inhibiting the activity of protein-tyrosine kinase; tyrphostin or pyrymidylaminobenzamide and derivatives thereof, e.g. 4-Methyl-3-[[4-(3-pyridinyl)-2-pyrimidinyl]amino]-*N*-[5-(4-methyl-1H-imidazol-1-yl)-3-(trifluoromethyl)phenyl] benzamide. A tyrphostin is preferably a low molecular weight (Mᵣ <1500) compound, or a pharmaceutically acceptable salt thereof, especially a compound selected from the benzylidenemalonitrile class or the S-arylbenzenemalonirile or bisubstrate quinoline class of compounds, more especially any compound selected from the group consisting of Tyrphostin A23/RG-50810, AG 99, Tyrphostin AG 213, Tyrphostin AG 1748, Tyrphostin AG 490, Tyrphostin B44, Tyrphostin B44 (+) enantiomer, Tyrphostin AG 555, AG 494, Tyrphostin AG 556; AG957 and adaphostin (4-{[(2,5-dihydroxyphenyl)methyl]amino}-benzoic acid adamantyl ester, NSC 680410, adaphostin);
xiv) compounds targeting, decreasing or inhibiting the activity of the epidermal growth factor family of receptor tyrosine kinases (EGFR, ErbB2, ErbB3, ErbB4 as homo- or heterodimers), such as compounds which target, decrease or inhibit the activity of the epidermal growth factor receptor family are especially compounds, proteins or antibodies which inhibit members of the EGF receptor tyrosine kinase family, e.g., EGF receptor, ErbB2, ErbB3 and ErbB4 or bind to EGF or EGF-related ligands, and are in particular those compounds, proteins or monoclonal antibodies generically and specifically disclosed in WO 97/02266, e.g., the compound of Example 39, or in EP 0 564 409, WO 99/03854, EP 0520722, EP 0 566 226, EP 0 787 722, EP 0 837 063, U.S. Patent No. 5,747,498, WO 98/10767, WO 97/30034, WO 97/49688, WO 97/38983 and, especially, WO 96/30347, e.g. compound known as CP 358774, WO 96/33980, e.g. compound ZD 1839; and WO 95/03283, e.g., compound ZM105180, e.g., trastuzumab (HERCEPTIN^{®}), cetuximab, Iressa, OSI-774, CI-1033, EKB-569, GW-2016, E1.1, E2.4, E2.5, E6.2, E6.4, E2.11, E6.3 or E7.6.3, and 7H-pyrrolo-[2,3-d]pyrimidine derivatives which are disclosed in WO 03/013541, erlotinib and gefitinib. Erlotinib can be administered in the form as it is marketed, e.g. TARCEVA, and gefitinib as IRESSA, human monoclonal antibodies against the epidermal growth factor receptor including ABX-EGFR; and
xv) Compounds which target, decrease or inhibit the activity/function of serine/theronine mTOR kinase are especially compounds, proteins or antibodies which target/inhibit members of the mTOR kinase family, e.g., RAD, RAD001, CCI-779, ABT578, SAR543, rapamycin and derivatives/analogs thereof, AP23573 and AP23841 from Ariad, everolimus (CERTICAN) and sirolimus. CERTICAN (everolimus, RAD) an investigational novel proliferation signal inhibitor that prevents proliferation of T-cells and vascular smooth muscle cells.

When referring to antibody, it is to include intact monoclonal antibodies, nanobodies, polyclonal antibodies, multi-specific antibodies formed from at least 2 intact antibodies, and antibodies fragments so long as they exhibit the desired biological activity.

The phrase "compound which targets, decreases or inhibits the activity of a protein or lipid phosphatase" as used herein includes but is not limited to inhibitors of phosphatase 1, phosphatase 2A, PTEN or CDC25, e.g. okadaic acid or a derivative thereof.

The term "further anti-angiogenic compounds" as used herein relates to thalidomide, e.g. THALOMID™, lenalidomide, e.g. Revlimid®, TNP-470 and SU5416.

The phrase "compounds which induce cell differentiation processes" as used herein, include but is not limited to retinoic acid, tocopherol or tocotrienol.

The term "monoclonal antibodies", as used herein, includes, but is not limited to bevacizumab, cetuximab, trastuzumab, Ibritumomab tiuxetan, and tositumomab and iodine I 131. Bevacizumab can be administered in the form as it is marketed, e.g. AVASTIN; cetuximab as ERBITUX; trastuzumab as HERCEPTIN; Rituximab as MABTHERA; Ibritumomab tiuxetan as ZEVULIN; and tositumomab and iodine I 131 as BEXXAR.

The term "cyclooxygenase inhibitor" as used herein includes, but is not limited to, e.g., Cox-2 inhibitors, 5-alkyl substituted 2-arylaminophenylacetic acid and derivatives, such as celecoxib (CELEBREX), rofecoxib (VIOXX), etoricoxib, valdecoxib or a 5-alkyl-2-arylaminophenylacetic acid, e.g. 5-methyl-2-(2'-chloro-6'-fluoroanilino)phenyl acetic acid, lumiracoxib.

The term "bisphosphonates" as used herein includes, but is not limited to etridonic acid, clodronic acid, tiludronic acid, pamidronic acid, alendronic acid, ibandronic acid, risedronic acid and zoledronic acid. "Etridonic acid" can be administered, e.g. in the form as it is marketed, e.g. under the trademark DIDRONEL™. "Clodronic acid" can be administered, e.g. in the form as it is marketed, e.g. under the trademark BONEFOS™. "Tiludronic acid" can be administered, e.g. in the form as it is marketed, e.g. under the trademark SKELID™. "Pamidronic acid" can be administered, e.g. in the form as it is marketed, e.g. under the trademark AREDIA™. "Alendronic acid" can be administered, e.g. in the form as it is marketed, e.g. under the trademark FOSAMAX™. "Ibandronic acid" can be administered, e.g. in the form as it is marketed, e.g. under the trademark BONDRANAT™. "Risedronic acid" can be administered, e.g. in the form as it is marketed, e.g. under the trademark ACTONEL™. "Zoledronic acid" can be administered, e.g. in the form as it is marketed, e.g. under the trademark ZOMETA™.

The term "heparanase inhibitor", as used herein, refers to compounds which target, decrease or inhibit heparin sulphate degradation. The term includes, but is not limited to, PI-88.

The term "biological response modifier", as used herein, includes, but is not limited to lymphokine or interferons, e.g., interferon γ.

The term "inhibitor of Ras oncogenic isoforms", as used herein, includes, but is not limited to H-Ras, K-Ras or N-Ras, as used herein, refers to compounds which target, decrease or inhibit the oncogenic activity of Ras, e.g. a farnesyl transferase inhibitor (FTI), e.g. L-744832, DK8G557 or R115777 (ZARNESTRA).

The term "telomerase inhibitor", as used herein, includes, but is not limited to compounds which target, decrease or inhibit the activity of telomerase. Compounds which target, decrease or inhibit the activity of telomerase are especially compounds which inhibit the telomerase receptor, e.g. telomestatin.

The term "matrix metalloproteinase inhibitor" or (MMP inhibitor), as used herein, includes, but is not limited to, collagen peptidomimetic and non-peptidomimetic inhibitors; tetracycline derivatives, e.g., hydroxamate peptidomimetic inhibitor batimastat; and its orally-bioavailable analogue marimastat (BB-2516), prinomastat (AG3340), metastat (NSC 683551) BMS-279251, BAY 12-9566, TAA211, MMI270B or AAJ996.

The term "methionine aminopeptidase inhibitor", as used herein, includes, but is not limited to, compounds which target, decrease or inhibit the activity of methionine aminopeptidase. Compounds which target, decrease or inhibit the activity of methionine aminopeptidase are, e.g. bengamide or a derivative thereof.

The term "proteasome inhibitors", as used herein, includes compounds which target, decrease or inhibit the activity of the proteosome. Compounds which target, decrease or inhibit the activity of the proteosome include, but are not limited to, PS-341, MLN 341, bortezomib or Velcade.

The phrase "agent used in the treatment of hematologic malignancies", as used herein, includes, but is not limited to, FMS-like tyrosine kinase inhibitors, e.g., compounds targeting, decreasing or inhibiting the activity of FMS-like tyrosine kinase receptors (Flt-3R); interferon, 1-b-D-arabinofuransylcytosine (ara-c) and bisulfan; and ALK inhibitors, e.g. compounds which target, decrease or inhibit anaplastic lymphoma kinase.

The phrase "compounds which target, decrease or inhibit the activity of Flt-3" as used herein, includes, but is not limited to compounds, proteins or antibodies which inhibit Flt-3, e.g. N-benzoyl-staurosporine, midostaurin, a staurosporine derivative, SU11248 and MLN518.

The term "HSP90 inhibitors", as used herein, includes, but is not limited to, compounds targeting, decreasing or inhibiting the intrinsic ATPase activity of HSP90; degrading, targeting, decreasing or inhibiting the HSP90 client proteins via the ubiquitin proteosome pathway. Compounds targeting, decreasing or inhibiting the intrinsic ATPase activity of HSP90 are especially compounds, proteins or antibodies which inhibit the ATPase activity of HSP90, e.g. 17-allylamino,17-demethoxygeldanamycin (17AAG), a geldanamycin derivative; other geldanamycin-related compounds; radicicol and HDAC inhibitors.

The term "anti-proliferative antibodies" as used herein, includes, but is not limited to trastuzumab (HERCEPTIN), trastuzumab-DM1, erlotinib (TARCEVA), bevacizumab (AVASTIN), rituximab (RITUXAN), PRO64553 (anti-CD40) and 2C4 Antibody. By antibodies is meant e.g. intact monoclonal antibodies, polyclonal antibodies, multispecific antibodies formed from at least 2 intact antibodies, and antibodies fragments so long as they exhibit the desired biological activity.

The term "histone deacetylase (HDAC) inhibitor", as used herein relates to relates to compounds which inhibit the histone deacetylase and which possesses anti-proliferative activity. This includes but is not limited to compounds disclosed in WO 02/22577, especially N-hydroxy-3-[4-[[(2-hydroxyethyl)[2-(1H-indol-3-yl)ethyl]-amino]methyl]phenyl]-2E-2-propenamide, and N-hydroxy-3-[4-[[[2-(2-methyl-1H-indol-3-yl)-ethyl]-amino]methyl]phenyl]-2E-2-propenamide and pharmaceutically acceptable salts thereof. It further especially includes Suberoylanilide hydroxamic acid (SAHA); MS-275, FK228 (formerly FR901228), [4-(2-amino-phenylcarbamoyl)-benzyl]-carbamic acid pyridine-3-ylmethyl ester and derivatives thereof; butyric acid, pyroxamide, trichostatin A, Oxamflatin, apicidin, depsipeptide, depudecin, trapoxin and the compounds disclosed in WO 02/22577, in particular N-hydroxy-3-[4-({(2-hydroxy-ethyl)-[2-(1H-indol-3-yl)-ethyl]-amino}-methyl-phenyl]-acrylamide or its lactate salt.

The phrase "compound which targets, decreases or inhibits the activity/function of serine/threonine mTOR kinase" as used herein, includes but is not limited to compounds, proteins or antibodies which target/inhibit members of the mTOR kinase family, e.g. RAD, RAD001, CCI-779, ABT578, SAR543, rapamycin and derivatives/analogs thereof, AP23573 and AP23841 from Ariad, everolimus (CERTICAN) and sirolimus (RAPAMUNE), CCI-779 and ABT578. CERTICAN (everolimus, RAD) an investigational novel proliferation signal inhibitor that prevents proliferation of T-cells and vascular smooth muscle cells.

The term "somatostatin receptor antagonist", as used herein, includes, but is not limited to, agents which target, treat or inhibit the somatostatin receptor, such as octreotide, vapreotide, lanreotide, and SOM230.

The term "anti-leukemic compound" as used herein, includes, but is not limited to Ara-C, a pyrimidine analog, which is the 2'-α-hydroxy ribose (arabinoside) derivative of deoxycytidine. Also included is the purine analog of hypoxanthine, 6-mercaptopurine (6-MP) and fludarabine phosphate.

The phrase "tumor cell damaging approaches" refers to approaches, such as ionizing radiation. The term "ionizing radiation", referred to above and hereinafter, means ionizing radiation that occurs as either electromagnetic rays, such as X-rays and gamma rays; or particles, such as alpha, beta and gamma particles. Ionizing radiation is provided in, but not limited to, radiation therapy and is known in the art. See Hellman, Cancer, 4th Edition, Vol. 1, Devita et al., Eds., pp. 248-275 (1993).

The term "EDG binder" as used herein, includes, but is not limited to, a class of immunosuppressants that modulates lymphocyte recirculation, such as FTY720.

The term "ribonucleotide reductase inhibitor" as used herein, includes, but is not limited to, pyrimidine or purine nucleoside analogs including, but not limited to, fludarabine and/or ara-C; 6-thioguanine; 5-FU; cladribine; 6-mercaptopurine, especially in combination with ara-C against ALL; and/or pentostatin. Ribonucleotide reductase inhibitors are especially hydroxyurea or 2-hydroxy-*1H*-isoindole-1,3-dione derivatives, such as PL-1, PL-2, PL-3, PL-4, PL-5, PL-6, PL-7 or PL-8. See Nandy et al., Acta Oncologica, Vol. 33, No. 8, pp. 953-961 (1994).

The term "S-adenosylmethionine decarboxylase inhibitors" as used herein includes, but is not limited to the compounds disclosed in US 5,461,076.

The phrase "monoclonal antibodies of VEGF or VEGFR", as used herein, includes but is not limited to, compounds disclosed in WO 98/35958, e.g. 1-(4-chloroanilino)-4-(4-pyridylmethyl)phthalazine or a pharmaceutically acceptable salt thereof, e.g., the succinate, or in WO 00/09495, WO 00/27820, WO 00/59509, WO 98/11223, WO 00/27819 and EP 0 769 947; those as described by Prewett et al., Cancer Res, Vol. 59, pp. 5209-5218 (1999); Yuan et al., Proc Natl Acad Sci USA, Vol. 93, pp. 14765-14770 (1996); Zhu et al., Cancer Res, Vol. 58, pp. 3209-3214 (1998); and Mordenti et al., Toxicol Pathol, Vol. 27, No. 1, pp. 14-21 (1999) in WO 00/37502 and WO 94/10202; ANGIOSTATIN, described by O'Reilly et al., Cell, Vol. 79, pp. 315-328 (1994); ENDOSTATIN, described by O'Reilly et al., Cell, Vol. 88, pp. 277-285 (1997); anthranilic acid amides; ZD4190; ZD6474; SU5416; SU6668; bevacizumab; or anti-VEGF antibodies or anti-VEGF receptor antibodies, e.g., rhuMAb and RHUFab; VEGF aptamer, e.g., Macugon; FLT-4 inhibitors; FLT-3 inhibitors; VEGFR-2 IgG1 antibody; Angiozyme (RPI 4610); and Avastan.

The term "photodynamic therapy", as used herein, refers to therapy which uses certain chemicals known as photosensitizing agents to treat or prevent cancers. Examples of photodynamic therapy include, but are not limited to, treatment with agents, such as, e.g., VISUDYNE and porfimer sodium.

The term "angiostatic steroid", as used herein, includes, but is not limited to agents which block or inhibit angiogenesis, such as, e.g., anecortave, triamcinolone, hydrocortisone, 11-α-epihydrocotisol, cortexolone, 17α-hydroxyprogesterone, corticosterone, desoxycorticosterone, testosterone, estrone and dexamethasone.

The phrase "Implant containing corticosteroids" as used herein, includes, but is not limited to agents, such as, e.g. fluocinolone and dexamethasone.

The term "AT1 receptor antagonist" as used herein, includes, but is not limited to agents, such as DIOVAN.

The term "ACE inhibitor' as used herein, includes, but is not limited to CIBACEN, benazepril, enazepril (LOTENSIN), captopril, enalapril, fosinopril, lisinopril, moexipril, quinapril, ramipril, perindopril and trandolapril.

Other pharmaceutically active agents include, but are not limited to, plant alkaloids, hormonal agents and antagonists, biological response modifiers, preferably lymphokines or interferons, antisense oligonucleotides or oligonucleotide derivatives; or miscellaneous agents or agents with other or unknown mechanism of action.

In each case where citations of patent applications or scientific publications are given, in particular with regard to the respective compound claims and the final products of the working examples therein, the subject matter of the final products, the pharmaceutical preparations and the claims is hereby incorporated into the present application by reference to these publications. Comprised are likewise the corresponding stereoisomers, as well as the corresponding crystal modifications, e.g., solvates and polymorphs, which are disclosed therein. The compounds used as active ingredients in the combinations disclosed herein can be prepared and administered as described in the cited documents, respectively.

The structure of the active agents identified by code numbers, generic or trade names may be taken from the actual edition of the standard compendium "The Merck Index" or from databases, e.g. Patents International, e.g. IMS World Publications. The corresponding content thereof is hereby incorporated by reference.

In one embodiment, the invention relates to a combination of an iron chelator of the formula II, in which R₁ and R₅ simultaneously or independently of one another are hydrogen, halogen, lower alkyl, halo-lower alkyl, lower alkoxy or halo-lower alkoxy; R₂ and R₄ simultaneously or independently of one another are hydrogen or a radical which can be removed under physiological conditions; R₃ is R₆R₇N-C(O)-lower alkyl, substituted aryl, aryl-lower alkyl, substituted by N-lower alkylamino, N,N-di-lower alkyl amino or pyrrolidino, or unsubstituted or substituted heteroaralkyl with the proviso that R₃ is not phenyl, substituted by halogen, nitro, nitrile, hydroxyl, lower alkyl, halo-lower alkyl, lower alkoxy or lower alkoxycarbonyl, if R₂ and R₄ are hydrogen and R₁ and R₅ are hydrogen, halogen, lower alkyl, halo-lower alkyl or lower alkoxy; R₆ and R₇ simultaneously or independently of one another are hydrogen, lower alkyl, hydroxy-lower alkyl, alkoxy-lower alkyl, hydroxyalkoxy-lower alkyl, amino-lower alkyl, N-lower alkylamino-lower alkyl, N,N-di-lower alkylamino-lower alkyl, N-(hydroxy-lower alkyl)amino-lower alkyl, N,N-di(hydroxy-lower alkyl)amino-lower alkyl or, together with the nitrogen atom to which they are bonded, form an azaalicyclic ring; and salts thereof; and

### (b) one or more pharmaceutically antineoplastic agents.

In one embodiment, the invention relates to a combination of
(a) a compound of formula II 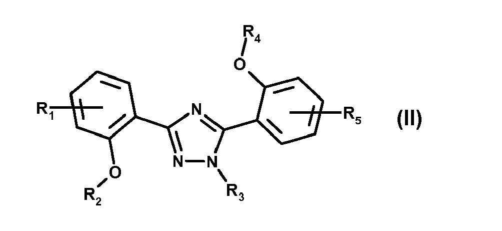 in which
   R₁ and R₅ simultaneously or independently of one another are hydrogen, halogen, lower-alkyl, halo-lower alkyl, lower alkoxy, halo-lower alkoxy, carboxyl, carbamoyl, N-lower alkylcarbamoyl, N,N-di-lower alkylcarbamoyl or nitrile; R₂ and R₄ simultaneously or independently of one another are hydrogen, unsubstituted or substituted lower alkanoyl or aroyl, or a radical which can be removed under physiological conditions; R₃ is R₆R₇N-C(O)-lower alkyl, unsubstituted or substituted aryl, aryl-lower alkyl, substituted by N-lower alkylamino, N,N-di-lower alkylamino or pyrrolidino, or unsubstituted or substituted heteroaryl or heteroaralkyl, with the proviso that R₃ is not phenyl or phenyl substituted by halogen, nitro, nitrile, hydroxyl, lower alkyl, halo-lower alkyl, lower alkoxy or lower alkoxycarbonyl if R₂ and R₄ are hydrogen, and R₁ and R₅ are hydrogen, halogen, lower alkyl, halo-lower alkyl, lower alkoxy or nitrile; R₆ and R₇ simultaneously or independently of one another are hydrogen, lower alkyl, hydroxy-lower alkyl, alkoxy-lower alkyl, hydroxyalkoxy-lower alkyl, amino-lower alkyl, N-lower alkylamino-lower alkyl, N,N-di-lower alkylamino-lower alkyl, N-(hydroxy-lower alkyl)amino-lower alkyl, N,N-di(hydroxy-lower alkyl)amino-lower alkyl or, together with the nitrogen atom to which they are bonded, form an azaalicyclic ring;
   and salts thereof; and
(b) one or more pharmaceutically active agents selected from the group consisting of an aromatase inhibitor; an antiestrogen; an anti-androgen; a gonadorelin agonist; a topoisomerase I inhibitor; a topoisomerase II inhibitor; a microtubule active agent; an alkylating agent; an anti-neoplastic anti-metabolite; a platin compound; a compound targeting/decreasing a protein or lipid kinase activity or a protein or lipid phosphatase activity, a anti-angiogenic compound; a compound which induces cell differentiation processes; monoclonal antibodies; a cyclooxygenase inhibitor; a bisphosphonate; a heparanase inhibitor; a biological response modifier; an inhibitor of Ras oncogenic isoforms; a telomerase inhibitor; a protease inhibitor, a matrix metalloproteinase inhibitor, a methionine aminopeptidase inhibitor; a proteasome inhibitor; agents which target, decrease or inhibit the activity of Flt-3; an HSP90 inhibitor; antiproliferative antibodies; an HDAC inhibitor; a compound which targets, decreases or inhibits the activity/function of serine/theronine mTOR kinase; a somatostatin receptor antagonist; an anti-leukemic compound; tumor cell damaging approaches; an EDG binder; a ribonucleotide reductase inhibitor; an S-adenosylmethionine decarboxylase inhibitor; a monoclonal antibody of VEGF or VEGFR; photodynamic therapy; an Angiostatic steroid; an implant containing corticosteroids; an AT1 receptor antagonist; and an ACE inhibitor.

The present invention relates to a method for the prevention of proliferative diseases or diseases that are triggered by persistent angiogenesis in a mammal, preferably a human patient, which comprises treating the patient concurrently or sequentially with pharmaceutically effective amounts of a combination of:
(a) an iron chelator; and
(b) an pharmaceutically active agent.

In another aspect, the present invention relates to a pharmaceutical composition comprising a combination of:
(a) an iron chelator; and
(b) a pharmaceutically active agent.

In a yet further aspect, the present invention provides a pharmaceutical preparation comprising:
(a) an iron chelator; and
(b) one or more pharmaceutically active agents, together with a pharmaceutically acceptable carrier.

In preferred embodiment, the present invention provides a pharmaceutical preparation comprising:
(a) a compound of formula II 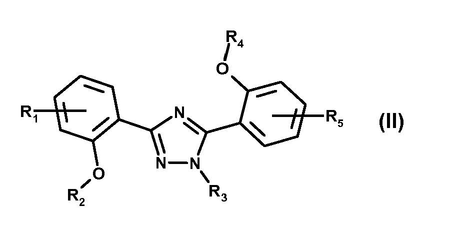 in which
   R₁ and R₅ simultaneously or independently of one another are hydrogen, halogen, lower-alkyl, halo-lower alkyl, lower alkoxy, halo-lower alkoxy, carboxyl, carbamoyl, N-lower alkylcarbamoyl, N,N-di-lower alkylcarbamoyl or nitrile; R₂ and R₄ simultaneously or independently of one another are hydrogen, unsubstituted or substituted lower alkanoyl or aroyl, or a radical which can be removed under physiological conditions; R₃ is R₆R₇N-C(O)-lower alkyl, unsubstituted or substituted aryl, aryl-lower alkyl, substituted by N-lower alkylamino, N,N-di-lower alkylamino or pyrrolidino, or unsubstituted or substituted heteroaryl or heteroaralkyl, with the proviso that R₃ is not phenyl or phenyl substituted by halogen, nitro, nitrile, hydroxyl, lower alkyl, halo-lower alkyl, lower alkoxy or lower alkoxycarbonyl if R₂ and R₄ are hydrogen, and R₁ and R₅ are hydrogen, halogen, lower alkyl, halo-lower alkyl, lower alkoxy or nitrile; R₆ and R₇ simultaneously or independently of one another are hydrogen, lower alkyl, hydroxy-lower alkyl, alkoxy-lower alkyl, hydroxyalkoxy-lower alkyl, amino-lower alkyl, N-lower alkylamino-lower alkyl, N,N-di-lower alkylamino-lower alkyl, N-(hydroxy-lower alkyl)amino-lower alkyl, N,N-di(hydroxy-lower alkyl)amino-lower alkyl or, together with the nitrogen atom to which they are bonded, form an azaalicyclic ring;
   and salts thereof; and
(b) one or more pharmaceutically active agents selected from the group consisting of an aromatase inhibitor; an antiestrogen; an anti-androgen; a gonadorelin agonist; a topoisomerase I inhibitor; a topoisomerase II inhibitor; a microtubule active agent; an alkylating agent; an anti-neoplastic anti-metabolite; a platin compound; a compound targeting/decreasing a protein or lipid kinase activity or a protein or lipid phosphatase activity, a anti-angiogenic compound; a compound which induces cell differentiation processes; monoclonal antibodies; a cyclooxygenase inhibitor; a bisphosphonate; a heparanase inhibitor; a biological response modifier; an inhibitor of Ras oncogenic isoforms; a telomerase inhibitor; a protease inhibitor, a matrix metalloproteinase inhibitor, a methionine aminopeptidase inhibitor; a proteasome inhibitor; agents which target, decrease or inhibit the activity of Flt-3; an HSP90 inhibitor; antiproliferative antibodies; an HDAC inhibitor; a compound which targets, decreases or inhibits the activity/function of serine/theronine mTOR kinase; a somatostatin receptor antagonist; an anti-leukemic compound; tumor cell damaging approaches; an EDG binder; a ribonucleotide reductase inhibitor; an S-adenosylmethionine decarboxylase inhibitor; a monoclonal antibody of VEGF or VEGFR; photodynamic therapy; an Angiostatic steroid; an implant containing corticosteroids; an AT1 receptor antagonist; and an ACE inhibitor.

In another preferred embodiment, the present invention provides a pharmaceutical preparation comprising:
(a) a compound of formula II 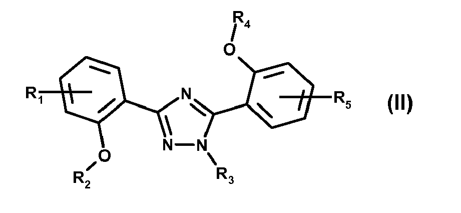 in which
   R₁ and R₅ simultaneously or independently of one another are hydrogen, halogen, lower-alkyl, halo-lower alkyl, lower alkoxy, halo-lower alkoxy, carboxyl, carbamoyl, N-lower alkylcarbamoyl, N,N-di-lower alkylcarbamoyl or nitrile; R₂ and R₄ simultaneously or independently of one another are hydrogen, unsubstituted or substituted lower alkanoyl or aroyl, or a radical which can be removed under physiological conditions; R₃ is R₆R₇N-C(O)-lower alkyl, unsubstituted or substituted aryl, aryl-lower alkyl, substituted by N-lower alkylamino, N,N-di-lower alkylamino or pyrrolidino, or unsubstituted or substituted heteroaryl or heteroaralkyl, with the proviso that R₃ is not phenyl or phenyl substituted by halogen, nitro, nitrile, hydroxyl, lower alkyl, halo-lower alkyl, lower alkoxy or lower alkoxycarbonyl if R₂ and R₄ are hydrogen, and R₁ and R₅ are hydrogen, halogen, lower alkyl, halo-lower alkyl, lower alkoxy or nitrile; R₆ and R₇ simultaneously or independently of one another are hydrogen, lower alkyl, hydroxy-lower alkyl, alkoxy-lower alkyl, hydroxyalkoxy-lower alkyl, amino-lower alkyl, N-lower alkylamino-lower alkyl, N,N-di-lower alkylamino-lower alkyl, N-(hydroxy-lower alkyl)amino-lower alkyl, N,N-di(hydroxy-lower alkyl)amino-lower alkyl or, together with the nitrogen atom to which they are bonded, form an azaalicyclic ring;
   and salts thereof; and
(b) one or more pharmaceutically active agents selected from the group consisting of a topoisomerase I inhibitor; a topoisomerase II inhibitor; a microtubule active agent; an alkylating agent; an anti-neoplastic anti-metabolite; a platin compound; a compound targeting/decreasing a protein or lipid kinase activity or a protein or lipid phosphatase activity, and an anti-angiogenic compound.

Any of the combination of components (a) and (b), the method of treating a warm-blooded animal comprising administering these two components, a pharmaceutical composition comprising these two components for simultaneous, separate or sequential use, the use of the combination for the delay of progression or the treatment of a proliferative disease or for the manufacture of a pharmaceutical preparation for these purposes or a commercial product comprising such a combination of components (a) and (b), all as mentioned or defined above, will be referred to subsequently also as COMBINATION OF THE INVENTION (so that this term refers to each of these embodiments which thus can replace this term where appropriate).

Simultaneous administration may, e.g., take place in the form of one fixed combination with two or more active ingredients, or by simultaneously administering two or more active ingredients that are formulated independently. Sequential use (administration) preferably means administration of one (or more) components of a combination at one time point, other components at a different time point, that is, in a chronically staggered manner, preferably such that the combination shows more efficiency than the single compounds administered independently (especially showing synergism). Separate use (administration) preferably means administration of the components of the combination independently of each other at different time points, preferably meaning that the components (a) and (b) are administered such that no overlap of measurable blood levels of both compounds are present in an overlapping manner (at the same time).

Also combinations of two or more of sequential, separate and simultaneous administration are possible, preferably such that the combination component-drugs show a joint therapeutic effect that exceeds the effect found when the combination component-drugs are used independently at time intervals so large that no mutual effect on their therapeutic efficiency can be found, a synergistic effect being especially preferred.

The term "delay of progression" as used herein means administration of the combination to patients being in a pre-stage or in an early phase, of the first manifestation or a relapse of the disease to be treated, in which patients, e.g., a pre-form of the corresponding disease is diagnosed or which patients are in a condition, e.g., during a medical treatment or a condition resulting from an accident, under which it is likely that a corresponding disease will develop.

"Jointly therapeutically active" or "joint therapeutic effect" means that the compounds may be given separately (in a chronically staggered manner, especially a sequence-specific manner) in such time intervals that they preferably, in the warm-blooded animal, especially human, to be treated, still show a (preferably synergistic) interaction (joint therapeutic effect). Whether this is the case, can *inter alia* be determined by following the blood levels, showing that both compounds are present in the blood of the human to be treated at least during certain time intervals.

"Pharmaceutically effective" preferably relates to an amount that is therapeutically or in a broader sense also prophylactically effective against the progression of a proliferative disease.

The term "a commercial package" or "a product", as used herein defines especially a "kit of parts" in the sense that the components (a) and (b) as defined above can be dosed independently or by use of different fixed combinations with distinguished amounts of the components (a) and (b), i.e., simultaneously or at different time points. Moreover, these terms comprise a commercial package comprising (especially combining) as active ingredients components (a) and (b), together with instructions for simultaneous, sequential (chronically staggered, in time-specific sequence, preferentially) or (less preferably) separate use thereof in the delay of progression or treatment of a proliferative disease. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the combination partners (a) and (b) (as can be determined according to standard methods. The ratio of the total amounts of the combination partner (a) to the combination partner (b) to be administered in the combined preparation can be varied, e.g., in order to cope with the needs of a patient sub-population to be treated or the needs of the single patient which different needs can be due to the particular disease, age, sex, body weight, etc. of the patients. Preferably, there is at least one beneficial effect, e.g., a mutual enhancing of the effect of the combination partners (a) and (b), in particular a more than additive effect, which hence could be achieved with lower doses of each of the combined drugs, respectively, than tolerable in the case of treatment with the individual drugs only without combination, producing additional advantageous effects, e.g., less side effects or a combined therapeutic effect in a non-effective dosage of one or both of the combination partners (components) (a) and (b), and very preferably a strong synergism of the combination partners (a) and (b).

Both in the case of the use of the combination of components (a) and (b) and of the commercial package, any combination of simultaneous, sequential and separate use is also possible, meaning that the components (a) and (b) may be administered at one time point simultaneously, followed by administration of only one component with lower host toxicity either chronically, e.g., more than 3-4 weeks of daily dosing, at a later time point and subsequently the other component or the combination of both components at a still later time point (in subsequent drug combination treatment courses for an optimal anti-tumor effect) or the like.

The COMBINATION OF THE INVENTION can also be applied in combination with other treatments, e.g., surgical intervention, hyperthermia and/or irradiation therapy.

The pharmaceutical compositions according to the present invention can be prepared by conventional means and are those suitable for enteral, such as oral or rectal, and parenteral administration to mammals including man, comprising a therapeutically effective amount of a VEGF inhibitor and at least one pharmaceutically active agent alone or in combination with one or more pharmaceutically acceptable carriers, especially those suitable for enteral or parenteral application.

The pharmaceutical compositions comprise from about 0.00002 to about 100%, especially, e.g., in the case of infusion dilutions that are ready for use) of 0.0001 to 0.02%, or, e.g., in case of injection or infusion concentrates or especially parenteral formulations, from about 0.1 % to about 95%, preferably from about 1% to about 90%, more preferably from about 20% to about 60% active ingredient (weight by weight, in each case). Pharmaceutical compositions according to the invention may be, e.g., in unit dose form, such as in the form of ampoules, vials, dragées, tablets, infusion bags or capsules.

The effective dosage of each of the combination partners employed in a formulation of the present invention may vary depending on the particular compound or pharmaceutical compositions employed, the mode of administration, the condition being treated and the severity of the condition being treated. A physician, clinician or veterinarian of ordinary skill can readily determine the effective amount of each of the active ingredients necessary to prevent, treat or inhibit the progress of the condition.

The term "a combined preparation", as used herein defines especially a "kit of parts" in the sense that the combination partners (a) and (b) as defined above can be dosed independently or by use of different fixed combinations with distinguished amounts of the combination partners (a) and (b), i.e., simultaneously or at different time points. The parts of the kit of parts can then, e.g., be administered simultaneously or chronologically staggered, that is at different time points and with equal or different time intervals for any part of the kit of parts. Thus, the present invention further includes a commercial package comprising a substituted 3,5-diphenyl-1,2,4-triazoles, e.g. 4-[3,5-bis(2-hydroxyphenyl)-[1,2,4]triazol-1-yl]benzoic acid or a pharmaceutically acceptable salt thereof in a form suitable for oral administration and instructions to administer the substituted 3,5-diphenyl-1,2,4-triazoles, e.g. 4-[3,5-bis(2-hydroxyphenyl)-[1,2,4]triazol-1-yl]benzoic acid or a pharmaceutically acceptable salt thereof while a patient is undergoing chemotherapy for a solid tumor disease.

The present invention also relates to the use of a COMBINATION OF THE INVENTION for the treatment of a proliferative disease and for the preparation of a medicament for the treatment of a proliferative disease.

The inventive combination therapy is especially useful for the treatment of liquid tumor diseases. The term "liquid tumor disease" means for example Acute lymphoblastic leukemia (ALL), acute myelogenous leukemia (AML), chronic myelogenous leukemia (CML), chronic lymphocytic leukemia (CLL), lymphoma, multiple myeloma and myelodysplastic syndrome.

The inventive combination therapy is especially useful for the treatment of solid tumor diseases. The term "solid tumor diseases" especially means liver cancer, e.g. hepocellular carcinoma, renal cancer, breast cancer, ovarian cancer, cancer of the colon, for example advances colorectal cancer, and generally the GI tract like, e.g. gastric cancer, cervix cancer, lung cancer, in particular small-cell lung cancer, and non-small-cell lung cancer, head and neck cancer, bladder cancer, cancer of the prostate, Kaposi's sarcoma, carcinoid tumors and carcinoid syndrome. The present combination inhibits the growth of solid tumors and liquid tumors and is also suited to prevent the metastatic growth of these tumors.

The term "carcinoid tumor" as used herein relates to a neuroendocrine tumor arising from the enterochromaffin cells which cells are scattered mainly throughout the intestine and main bronchi. Peptides synthesized by carcinoid tumors include 5-hydroxytryptamine and 5-hydroxytrypthophan.

The term "carcinoid syndrome" as used herein relates to a disease, in particluar the manifestation of an advanced disease, the symptoms of which include cutaneous flushing, diarrhea and palpable abdominal mass or hepatomegaly. In said disease the urinary concentration of 5-hydroxyindolacetic acid (5-HIAA) typically relates directly to the tumor volume and correlates with the chance of survival. A level of > 8 mg/24 hours of 5-HIAA is a sensitive measurement in 75 % of all cases of carcinoid syndrome. Another indicator for the syndrome is an increased plasma serotonin level, in particular a plasma serotonin level higher than about 250, especially 350 ng/ml.

The term "metastatic growth" as used herein comprises the metastatic spread of tumors and the growth and development of micrometastases in other organs of the cancer patients.

It will be understood that references to the combination partners (a) and (b) are meant to also include the pharmaceutically acceptable salts of the compounds.

A combination which comprises (a) at least one anti-neoplastic agent known in chemotherapy and (b) a substituted 3,5-diphenyl-1,2,4-triazole, e.g. 4-[3,5-bis(2-hydroxyphenyl)-[1,2,4]triazol-1-yl]benzoic acid, in which the active ingredients are present in each case in free form or in the form of a pharmaceutically acceptable salt and optionally at least one pharmaceutically acceptable carrier, will be referred to hereinafter as a COMBINATION OF THE INVENTION.

The nature of proliferative diseases is multifactorial. Under certain circumstances, drugs with different mechanisms of action may be combined. However, just considering any combination of drugs having different mode of action does not necessarily lead to combinations with advantageous effects.

All the more surprising is the experimental finding that *in vivo* the administration of a COMBINATION OF THE INVENTION, results not only in a beneficial effect, especially a synergistic therapeutic effect, e.g. with regard to slowing down, arresting or reversing the neoplasm formation or a longer duration of tumor response, but also in further surprising beneficial effects, e.g. less side-effects, an improved quality of life and a decreased mortality and morbidity, compared to a monotherapy applying only one of the pharmaceutically active ingredients used in the COMBINATION OF THE INVENTION.

The effective dosage of each of the combination partners employed in the COMBINATION OF THE INVENTION may vary depending on the particular compound or pharmaceutical composition employed, the mode of administration, the condition being treated, the severity of the condition being treated. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites.

The dosage of the active ingredient can depend on various factors, such as activity and duration of action of the active ingredient, severity of the illness to be treated or its symptoms, manner of administration, warm-blooded animal species, sex, age, weight and/or individual condition of the warm-blooded animal. The doses to be administered daily in the case of oral administration are between 10 and approximately 120 mg/kg, in particular 20 and approximately 80 mg/kg, and for a warm-blooded animal having a body weight of approximately 40 kg, preferably between approximately 400 mg and approximately 4,800 mg, in particular approximately 800 mg to 3,200 mg, which is expediently divided into 2 to 12 individual doses. However, other administration schedules are also likely to be effective and are included within the scope of the present invention.

In a COMBINATION OF THE INVENTION, the substituted 3,5-diphenyl-1,2,4-triazoles, e.g. 4-[3,5-bis(2-hydroxyphenyl)-[1,2,4]triazol-1-yl]benzoic acid or a pharmaceutically acceptable salt thereof, can be given on a continuous basis, for example daily, during and subsequent to the chemotherapy. A daily oral dose in the range from 500 mg to 4000 mg, for example in the range from 500 mg/day to 2000 mg/day, in particular 1000, 1500 or 2000 mg/day, are contemplated. However, other administration schedules are also included within the scope of the present invention. When the substituted 3,5-diphenyl-1,2,4-triazole is administered as a salt form, the above daily oral dosage ranges are adjusted so that an equivalent amount of free base administered.

When the combination partners employed in the COMBINATION OF THE INVENTION are applied in the form as marketed as single drugs, their dosage and mode of administration can take place in accordance with the information provided on the package insert of the respective marketed drug in order to result in the beneficial effect described herein, if not mentioned herein otherwise.

Carboplatin may be administered intravenously to a human in a dosage range varying from about 100 to 400, e.g. 200, mg/m² body surface about every four to six weeks.

Oxaliplatin may be administered intravenously to a human in a dosage range varying from about 25 to 135, e.g. 45 or 85, mg/m² body surface about every two to three weeks.

Cisplatin may be administered to a human in a dosage range varying from about 25 to 100 mg/m² about every three weeks.

Topotecan may be administered to a human in a dosage range varying from about 1 to 5 mg/m²day.

Irinotecan may be administered to a human in a dosage range varying from about 50 to 350 mg/m²day.

Fadrozole may be administered orally to a human in a dosage range varying from about 0.5 to about 10 mg/day, preferably from about 1 to about 2.5 mg/day

Exemestane may be administered orally to a human in a dosage range varying from about 5 to about 200 mg/day, preferably from about 10 to about 25 mg/day, or parenterally from about 50 to 500 mg/day, preferably from about 100 to about 250 mg/day. If the drug shall be administered in a separate pharmaceutical composition, it can be administered in the form disclosed in GB 2,177,700.

Formestane may be administered parenterally to a human in a dosage range varying from about 100 to 500 mg/day, preferably from about 250 to about 300 mg/day.

Anastrozole may be administered orally to a human in a dosage range varying from about 0.25 to 20 mg/day, preferably from about 0.5 to about 2.5 mg/day.

Aminogluthemide may be administered to a human in a dosage range varying from about 200 to 500 mg/day.

Tamoxifen citrate may be administered to a human in a dosage range varying from about 10 to 40 mg/day.

Vinblastine may be administered to a human in a dosage range varying from about 1.5 to 10 mg/m²/day.

Vincristine sulfate may be administered parenterally to a human in a dosage range varying from about 0.025 to 0.05 mg/kg body weight * week.

Vinorelbine may be administered to a human in a dosage range varying from about 10 to 50 mg/m²/day.

Etoposide phosphate may be administered to a human in a dosage range varying from about 25 to 115 mg/m²/day, e.g. 56.8 or 113.6 mg/m²/day.

Teniposide may be administered to a human in a dosage range varying from about 75 to 150 mg about every two weeks.

Doxorubicin may be administered to a human in a dosage range varying from about 10 to 100 mg/m²/day, e.g. 25 or 75 mg/m²day.

Epirubicin may be administered to a human in a dosage range varying from about 10 to 200 mg/m²/day.

Idarubicin may be administered to a human in a dosage range varying from about 0.5 to 50 mg/m²day, e.g. 8 mg/m²/day during three days.

Mitoxantrone may be administered to a human in a dosage range varying from about 2.5 to 25 mg/m²/day.

Paclitaxel may be administered to a human in a dosage range varying from about 50 to 300 mg/m²/day.

Alendronic acid may be administered to a human in a dosage range varying from about 5 to 10 mg/day.

Clodronic acid may be administered to a human e.g. in a dosage range varying from about 750 to 1500 mg/day.

Etridonic acid may be administered to a human in a dosage range varying from about 200 to 400 mg/day.

Ibandronic acid may be administered to a human in a dosage range varying from about 1 to 4 mg every three to four weeks.

Risedronic acid may be administered to a human in a dosage range varying from about 20 to 30 mg/day.

Pamidronic acid may be administered to a human in a dosage range varying from about 15 to 90 mg every three to four weeks.

Tiludronic acid may be administered to a human in a dosage range varying from about 200 to 400 mg/day.

Bicalutamide may be administered to a human in a dosage range varying from about 25 to 50 mg/m²day.

ADRIAMYCIN may be administered in the range from 100-1500 mg daily, e.g., 200-1000 mg/day, such as 200, 400, 500, 600, 800, 900 or 1000 mg/day, administered in one or two doses daily.

5-FU may be administered at a appropriate dose in the range from 100-1500 mg daily, e.g. 200-1000 mg/day, such as 200, 400, 500, 600, 800, 900 or 1000 mg/day, administered in one or two doses daily.

CAMPTOTHECIN may be administered at a appropriate dose in the range from 100-1500 mg daily, e.g., 200-1000 mg/day, such as 200, 400, 500, 600, 800, 900 or 1000 mg/day, administered in one or two doses daily.

5-AZACYTIDINE may be administered at a appropriate dose in the range from 100-1500 mg daily, e.g., 200-1000 mg/day, such as 200, 400, 500, 600, 800, 900 or 1000 mg/day, administered in one or two doses daily.

TOMUDEX (raltitrexed, AstraZeneca Pharmaceuticals, Wilmington DE, USA) can be administered according tot manufacturer's instructions.

REVLIMID, known as lenalidomide and CC-5013 is marketed by Celgene and is a derivative of thalidomide introduced in 2004. It was initially intended as a treatment for multiple myeloma, for which thalidomide is an accepted therapeutic modality, but has also shown efficacy in the hematological disorders known as the myelodysplastic syndromes.

Tyrphostins, especially Adaphostin, are preferably administered to a warm-blooded animal, especially a human in a dosage in the range of about 1-6000 mg/day, more preferably 25-5000 mg/day, most preferably 50-4000 mg/day. Unless stated otherwise herein, the compound is preferably administered from one to 5, especially from 1-4 times per day.

Pharmaceutical preparations for the combination therapy for enteral or parenteral administration are, e.g., those in unit dosage forms, such as sugar-coated tablets, capsules or suppositories, and furthermore ampoules. If not indicated otherwise, these formulations are prepared by conventional means, e.g., by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units. One of skill in the art has the ability to determine appropriate pharmaceutically effective amounts of the combination components.

Preferably, the compounds or the pharmaceutically acceptable salts thereof, are administered as an oral pharmaceutical formulation in the form of a tablet, capsule or syrup; or as parenteral injections if appropriate.

In preparing compositions for oral administration, any pharmaceutically acceptable media may be employed such as water, glycols, oils, alcohols, flavoring agents, preservatives, coloring agents. Pharmaceutically acceptable carriers include starches, sugars, microcrystalline celluloses, diluents, granulating agents, lubricants, binders, disintegrating agents.

Solutions of the active ingredient, and also suspensions, and especially isotonic aqueous solutions or suspensions, are useful for parenteral administration of the active ingredient, it being possible, e.g., in the case of lyophilized compositions that comprise the active ingredient alone or together with a pharmaceutically acceptable carrier, e.g., mannitol, for such solutions or suspensions to be produced prior to use. The pharmaceutical compositions may be sterilized and/or may comprise excipients, e.g., preservatives, stabilizers, wetting and/or emulsifying agents, solubilizers, salts for regulating the osmotic pressure and/or buffers, and are prepared in a manner known per se, e.g., by means of conventional dissolving or lyophilizing processes. The solutions or suspensions may comprise viscosity-increasing substances, such as sodium carboxymethylcellulose, carboxymethylcellulose, dextran, polyvinylpyrrolidone or gelatin. Suspensions in oil comprise as the oil component the vegetable, synthetic or semi-synthetic oils customary for injection purposes.

The isotonic agent may be selected from any of those known in the art, e.g. mannitol, dextrose, glucose and sodium chloride. The infusion formulation may be diluted with the aqueous medium. The amount of aqueous medium employed as a diluent is chosen according to the desired concentration of active ingredient in the infusion solution. Infusion solutions may contain other excipients commonly employed in formulations to be administered intravenously such as antioxidants.

The present invention further relates to "a combined preparation", which, as used herein, defines especially a "kit of parts" in the sense that the combination partners (a) and (b) as defined above can be dosed independently or by use of different fixed combinations with distinguished amounts of the combination partners (a) and (b), i.e., simultaneously or at different time points. The parts of the kit of parts can then, e.g., be administered simultaneously or chronologically staggered, that is at different time points and with equal or different time intervals for any part of the kit of parts. The ratio of the total amounts of the combination partner (a) to the combination partner (b) to be administered in the combined preparation can be varied, e.g., in order to cope with the needs of a patient sub-population to be treated or the needs of the single patient based on the severity of any side effects that the patient experiences.

The present invention especially relates to a combined preparation which comprises:
(a) one or more unit dosage forms of an iron chelator; and
(b) one or more unit dosage forms of an pharmaceutically active agent.

It is one objective of this invention to provide a pharmaceutical composition comprising a quantity, which is jointly therapeutically effective against a proliferative disease comprising the COMBINATION OF THE INVENTION. In this composition, the combination partners (a) and (b) can be administered together, one after the other or separately in one combined unit dosage form or in two separate unit dosage forms. The unit dosage form may also be a fixed combination.

The pharmaceutical compositions for separate administration of the combination partners (a) and (b) and for the administration in a fixed combination, i.e. a single galenical compositions comprising at least two combination partners (a) and (b), according to the invention can be prepared in a manner known per se and are those suitable for enteral, such as oral or rectal, and parenteral administration to mammals (warm-blooded animals), including man, comprising a therapeutically effective amount of at least one pharmacologically active combination partner alone or in combination with one or more pharmaceutically acceptable carries, especially suitable for enteral or parenteral application.

Novel pharmaceutical composition contain, for example, from about 10 % to about 100 %, preferably from about 20 % to about 60 %, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

Pharmaceutical composition comprising substituted 3,5-diphenyl-1,2,4-triazoles or pharmaceutically acceptable salts thereof are described in WO97/49395 published on December 31, 1997 and in the US granted patent 6,465,504. Dispersible tablets comprising 4-[3,5-bis(2-hydroxyphenyl)-[1,2,4]triazol-1-yl]benzoic acid are described in the International Patent Applications WO200/035026 published on April 29, 2004 and WO2005/097062 published on October 20, 2005.

A further aspect of the present invention relates to the use of improved regimens for the administration of substituted 3,5-diphenyl-1,2,4-triazoles, e.g. 4-[3,5-bis(2-hydroxyphenyl)-[1,2,4]triazol-1-yl]benzoic acid, or a pharmaceutically acceptable salt thereof, for the treatment of patients suffering from solid tumor diseases, including, e.g. liver cancer and, in particular, hepocellular carcinoma.

According to one inventive regimen substituted 3,5-diphenyl-1,2,4-triazoles, e.g. 4-[3,5-bis(2-hydroxyphenyl)-[1,2,4]triazol-1-yl]benzoic acid, or a pharmaceutically acceptable salt thereof, is administered twice or more daily, for example two or three times daily, in reduced amounts compared with the known once daily administration regimens. Alternatively, the present invention embraces a treatment regimen wherein substituted 3,5-diphenyl-1,2,4-triazoles, e.g. 4-[3,5-bis(2-hydroxyphenyl)-[1,2,4]triazol-1-yl]benzoic acid is administered once daily at a dose in the range from 50 mg/day to 4200 mg/day, particularly a dose of 1200 mg/day to 1300 mg/day, especially 1250 mg/day.

According to one aspect of the present invention, substituted 3,5-diphenyl-1,2,4-triazoles, e.g. 4-[3,5-bis(2-hydroxyphenyl)-[1,2,4]triazol-1-yl]benzoic acid is given daily alone, or during and subsequent to other therapies, for example chemotherapy. A daily oral administration of an amount in the range from 300 mg to 4000 mg, for example in the range from 300 mg/day to 2000 mg/day or 300 mg/day to 1000 mg/day, in particular 300, 500, 750, 1000, 1500 or 2000 mg/day, is contemplated as a pharmaceutically effective amount in the daily regimen.

When substituted 3,5-diphenyl-1,2,4-triazoles, e.g. 4-[3,5-bis(2-hydroxyphenyl)-[1,2,4]triazol-1-yl]benzoic acid is administered as a pharmaceutically acceptable salt, an equivalent amount of the free base (i.e. one equivalent to the amounts described above) is administered. In this application, reference to substituted 3,5-diphenyl-1,2,4-triazoles, e.g. 4-[3,5-bis(2-hydroxyphenyl)-[1,2,4]triazol-1-yl]benzoic acid is intended to include pharmaceutically acceptable salts thereof.

The different aspects of the present invention can be combined freely with each other. For example, a renal cancer patient or a patient having another tumor type can be treated by administering substituted 3,5-diphenyl-1,2,4-triazoles, e.g. 4-[3,5-bis(2-hydroxyphenyl)-[1,2,4]triazol-1-yl]benzoic acid on a daily basis or twice daily. The improved regimens of administering substituted 3,5-diphenyl-1,2,4-triazoles, e.g. 4-[3,5-bis(2-hydroxyphenyl)-[1,2,4]triazol-1-yl]benzoic acid can be applied in monotherapy or in combination therapy, with other words, substituted 3,5-diphenyl-1,2,4-triazoles, e.g. 4-[3,5-bis(2-hydroxyphenyl)-[1,2,4]triazol-1-yl]benzoic acid is administered alone, or in combination with other therapeutic agents. As a combination therapy, it is administered on a once or twice daily basis as described herein and any other therapeutic agent or agents are administered according to its established administration regimen.

The present invention relates to a combination or a "combined preparation" comprising (a) a 4-[3,5-bis(2-hydroxyphenyl)-[1,2,4]triazol-1-yl]benzoic acid and (b) a platinum compound selected from the group consisting of carboplatin, cisplatin, oxaliplatin, strataplatin and ZD0473.

In a further embodiment, the present invention pertains to a combination or a "combined preparation" comprising (a) a 4-[3,5-bis(2-hydroxyphenyl)-[1,2,4]triazol-1-yl]benzoic acid and (b) a platinum compound selected from the group consisting of carboplatin, cisplatin, and oxaliplatin.

In another further embodiment, the present invention pertains to a combination or a "combined preparation" comprising (a) a 4-[3,5-bis(2-hydroxyphenyl)-[1,2,4]triazol-1-yl]benzoic acid and (b) cisplatin.

The present invention relates to a combination or a "combined preparation" comprising (a) a 4-[3,5-bis(2-hydroxyphenyl)-[1,2,4]triazol-1-yl]benzoic acid and (b) a topoisomerase I inhibitors selected from the group consisting of topotecan, irinotecan, camptothecin, e.g. 9-nitrocamptothecin and the macromolecular camptothecin conjugate PNU-166148.

The present invention relates to a combination or a "combined preparation" comprising (a) a 4-[3,5-bis(2-hydroxyphenyl)-[1,2,4]triazol-1-yl]benzoic acid and (b) an aromatase inhibitor selected from the group consisting of atamestane, exemestane, formestane, aminoglutethimide, roglethimide, pyridogluthethimide, trilostane, testolactone, ketokonazole, vorozole, fadrozole, anastrozole and letrozole .

The present invention relates to a combination or a "combined preparation" comprising (a) a 4-[3,5-bis(2-hydroxyphenyl)-[1,2,4]triazol-1-yl]benzoic acid and (b) an anti-estrogen selected from the group consisting of tamoxifen, fulvestrant, raloxifene and raloxifene hydrochloride. The present invention relates to a combination or a "combined preparation" comprising (a) a 4-[3,5-bis(2-hydroxyphenyl)-[1,2,4]triazol-1-yl]benzoic acid and (b) a topoisomerase II inhibitor selected from the group consisting of doxorubicin, including liposomal formulation, e.g. CAELYX™, daunorubicin, including liposomal formulation, e.g. DAUNOSOME™, epirubicin, idarubicin, mytomycin C, pirarubicin, nemorubicin, mitoxantrone, losoxantrone, etoposide and teniposide.

In one embodiment the invention pertains to the combination of or a "combined preparation" comprising (a) a 4-[3,5-bis(2-hydroxyphenyl)-[1,2,4]triazol-1-yl]benzoic acid and (b) mytomycin C.

The present invention relates to a combination or a "combined preparation" comprising (a) a 4-[3,5-bis(2-hydroxyphenyl)-[1,2,4]triazol-1-yl]benzoic acid and (b) a microtubule active agent selected from the group consisting of paclitaxel and docetaxel, vinblastine, vinblastine sulfate, vincristine, vincristine sulfate, vinorelbine, discodermolide, colchicines, and epothiloneA, epothilone B or derivatives thereof.

The present invention relates to a combination or a "combined preparation" comprising (a) a 4-[3,5-bis(2-hydroxyphenyl)-[1,2,4]triazol-1-yl]benzoic acid and (b) an anti-angiogenic compound selected from the group consisting of thalidomide, lenalidomide, TNP-470 and SU5416.

The present invention relates to a combination or a "combined preparation" comprising (a) a 4-[3,5-bis(2-hydroxyphenyl)-[1,2,4]triazol-1-yl]benzoic acid and (b) an anti-angiogenic compound selected from the group consisting of thalidomide, TNP-470 and SU5416.

The present invention relates to a combination or a "combined preparation" comprising (a) a 4-[3,5-bis(2-hydroxyphenyl)-[1,2,4]triazol-1-yl]benzoic acid and (b) a gonadorelin agonist selected from the group consisting of abarelix, goserelin and goserelin acetate.

The present invention relates to a combination or a "combined preparation" comprising (a) a 4-[3,5-bis(2-hydroxyphenyl)-[1,2,4]triazol-1-yl]benzoic acid and (b) bicalutamide.

The present invention relates to a combination or a "combined preparation" comprising (a) a 4-[3,5-bis(2-hydroxyphenyl)-[1,2,4]triazol-1-yl]benzoic acid and (b) a biphosphonate selected from the group consisting of etridonic acid, clodronic acid, tiludronic acid, pamidronic acid, alendronic acid, ibandronic acid, risedronic acid and zoledronic acid.

The present invention relates to a combination or a "combined preparation" comprising (a) a 4-[3,5-bis(2-hydroxyphenyl)-[1,2,4]triazol-1-yl]benzoic acid and (b) an alkylating agent selected from the group consisting of cyclophosphamide, ifosfamide, melphalan, nitrosourea (BCNU or Gliadel), and temozolamide (TEMODAR).

The present invention relates to a combination or a "combined preparation" comprising (a) a 4-[3,5-bis(2-hydroxyphenyl)-[1,2,4]triazol-1-yl]benzoic acid and (b) an anti-neoplastic anti-metabolite selected from the group consisting of 5-fluorouracil (5-FU), capecitabine, gemcitabine, 5-azacytidine, decitabine, methotrexate, edatrexate, pemetrexed, leucovorin and raltitrexed (TOMUDEX), preferably from the group consisting of 5-FU, leucovorin and ralitrexed.

In one embodiment the invention pertains to the combination of or a "combined preparation" comprising (a) a 4-[3,5-bis(2-hydroxyphenyl)-[1,2,4]triazol-1-yl]benzoic acid and (b) 5-fluorouracil (5-FU).

In one embodiment the invention pertains to the combination of or a "combined preparation" comprising (a) a 4-[3,5-bis(2-hydroxyphenyl)-[1,2,4]triazol-1-yl]benzoic acid and (b) leucovorin.

In one embodiment the invention pertains to the combination of or a "combined preparation" comprising (a) a 4-[3,5-bis(2-hydroxyphenyl)-[1,2,4]triazol-1-yl]benzoic acid and (b) and raltitrexed (TOMUDEX).

The present invention relates to a combination or a "combined preparation" comprising (a) a 4-[3,5-bis(2-hydroxyphenyl)-[1,2,4]triazol-1-yl]benzoic acid and (b) a compound selected from the group consisting of phosphatase 1, phosphatase 2A, PTEN and CDC25, e.g. okadaic acid or a derivative thereof.

The present invention relates to a combination or a "combined preparation" comprising (a) a 4-[3,5-bis(2-hydroxyphenyl)-[1,2,4]triazol-1-yl]benzoic acid and (b) a compound selected from the group consisting of thalidomide, e.g. THALOMID™, lenalidomide, e.g. Revlimid®, TNP-470 and SU5416.

The present invention relates to a combination or a "combined preparation" comprising (a) a 4-[3,5-bis(2-hydroxyphenyl)-[1,2,4]triazol-1-yl]benzoic acid and (b) a compound selected from the group consisting of thalidomide, e.g. THALOMID™, TNP-470 and SU5416.

The present invention relates to a combination or a "combined preparation" comprising (a) a 4-[3,5-bis(2-hydroxyphenyl)-[1,2,4]triazol-1-yl]benzoic acid and (b) an anti-proliferative antibodies selected from the group consisting of trastuzumab (HERCEPTIN), trastuzumab-DM1, erlotinib (TARCEVA), bevacizumab (AVASTIN), rituximab (RITUXAN), PRO64553 (anti-CD40) and 2C4 Antibody.

The present invention relates to a combination or a "combined preparation" comprising (a) a 4-[3,5-bis(2-hydroxyphenyl)-[1,2,4]triazol-1-yl]benzoic acid and (b) a histone deacetylase (HDAC) inhibitor selected from the group consistinf of N-hydroxy-3-[4-[[(2-hydroxyethyl)[2-(1H-indol-3-yl)ethyl]-amino]methyl]phenyl]-2E-2-propenamide, N-hydroxy-3-[4-[[[2-(2-methyl-1H-indol-3-yl)-ethyl]-amino]methyl]phenyl]-2E-2-propenamide, Suberoylanilide hydroxamic acid (SAHA), MS-275, FK228 (formerly FR901228), [4-(2-aminophenylcarbamoyl)-benzyl]-carbamic acid pyridine-3-ylmethyl ester, butyric acid, pyroxamide, trichostatin A, Oxamflatin, apicidin, Depsipeptide, depudecin, trapoxin, N-hydroxy-3-[4-({(2-hydroxy-ethyl)-[2-(1H-indol-3-yl)-ethyl]-amino}-methyl-phenyl]-acrylamide or a pharmaceutically acceptable salts thereof.

The present invention relates to a combination or a "combined preparation" comprising (a) a 4-[3,5-bis(2-hydroxyphenyl)-[1,2,4]triazol-1-yl]benzoic acid and (b) a compound selected from the group consisting of retinoic acid, tocopherol or tocotrienol.

The present invention relates to a combination or a "combined preparation" comprising (a) a 4-[3,5-bis(2-hydroxyphenyl)-[1,2,4]triazol-1-yl]benzoic acid and (b) PI-88.

The present invention relates to a combination or a "combined preparation" comprising (a) a 4-[3,5-bis(2-hydroxyphenyl)-[1,2,4]triazol-1-yl]benzoic acid and (b) FTY720.

The present invention relates to a combination or a "combined preparation" comprising (a) a 4-[3,5-bis(2-hydroxyphenyl)-[1,2,4]triazol-1-yl]benzoic acid and (b) a compound which targets, decreases or inhibits the activity/function of serine/threonine mTOR kinase selected from the group consisting of RAD, RAD001, CCI-779, ABT578, SAR543, rapamycin, rapamycin derivatives/analogs thereof, AP23573, AP23841, everolimus (CERTICAN) sirolimus (RAPAMUNE), CCI-779 and ABT578.

The present invention relates to a combination or a "combined preparation" comprising (a) a 4-[3,5-bis(2-hydroxyphenyl)-[1,2,4]triazol-1-yl]benzoic acid and (b) an HSP90 inhibitor selected from the group consisting of 17-allylamino,17-demethoxygeldanamycin (17AAG), geldanamycin derivatives, geldanamycin-related compounds and radicicol.

The present invention relates to a combination or a "combined preparation" comprising (a) a 4-[3,5-bis(2-hydroxyphenyl)-[1,2,4]triazol-1-yl]benzoic acid and (b) an anti-leukemic compound selected from the group consisting of Ara-C, 6-mercaptopurine (6-MP) and fludarabine phosphate.

The present invention relates to a combination or a "combined preparation" comprising (a) a 4-[3,5-bis(2-hydroxyphenyl)-[1,2,4]triazol-1-yl]benzoic acid and (b) a cyclooxygenase inhibitor selected from the group consisting of celecoxib (CELEBREX), rofecoxib (VIOXX), etoricoxib, valdecoxib, 5-methyl-2-(2'-chloro-6'-fluoroanilino)phenyl acetic acid, and lumiracoxib.

The present invention relates to a combination or a "combined preparation" comprising (a) a 4-[3,5-bis(2-hydroxyphenyl)-[1,2,4]triazol-1-yl]benzoic acid and (b) a compound selected from the group consisting of L-744832, DK8G557 and R115777 (ZARNESTRA).

The present invention relates to a combination or a "combined preparation" comprising (a) a 4-[3,5-bis(2-hydroxyphenyl)-[1,2,4]triazol-1-yl]benzoic acid and (b) a proteasome inhibitor selected from the group consisting of PS-341, MLN 341, bortezomib or Velcade.

The present invention relates to a combination or a "combined preparation" comprising (a) a 4-[3,5-bis(2-hydroxyphenyl)-[1,2,4]triazol-1-yl]benzoic acid and (b) DIOVAN.

The present invention relates to a combination or a "combined preparation" comprising (a) a 4-[3,5-bis(2-hydroxyphenyl)-[1,2,4]triazol-1-yl]benzoic acid and (b) telomestatin.

The present invention relates to a combination or a "combined preparation" comprising (a) a 4-[3,5-bis(2-hydroxyphenyl)-[1,2,4]triazol-1-yl]benzoic acid and (b) an ACE inhibitor selected from the group consisting of CIBACEN, benazepril, enazepril (LOTENSIN), captopril, enalapril, fosinopril, lisinopril, moexipril, quinapril, ramipril, perindopril and trandolapril.

The present invention relates to a combination or a "combined preparation" comprising (a) a 4-[3,5-bis(2-hydroxyphenyl)-[1,2,4]triazol-1-yl]benzoic acid and (b) a MMP inhibitor selected from the group consisting of batimastat, marimastat (BB-2516), prinomastat (AG3340), metastat (NSC 683551) BMS-279251, BAY 12-9566, TAA211, MMI270B and AAJ996.

The present invention relates to a combination or a "combined preparation" comprising (a) a 4-[3,5-bis(2-hydroxyphenyl)-[1,2,4]triazol-1-yl]benzoic acid and (b) bengamide or a derivative thereof.

The present invention relates to a combination or a "combined preparation" comprising (a) a 4-[3,5-bis(2-hydroxyphenyl)-[1,2,4]triazol-1-yl]benzoic acid and (b) a somatostatin receptor antagonist selected from the group consisting of pasireotide, lanreotide, octreotide and vapreotide.

The present invention relates to a combination or a "combined preparation" comprising (a) a 4-[3,5-bis(2-hydroxyphenyl)-[1,2,4]triazol-1-yl]benzoic acid and (b) a compound selected from the group consisting of (4-tert-butyl-phenyl)-94-pyridin-4-ylmethyl-isoquinolin-1-yl)-amino, SU101, SU6668, GFB-111, PD180970, AG957, NSC 680410, PD173955, BMS354825, UCN-01, safingol; BAY 43-9006, Bryostatin 1, Perifosine, Ilmofosine, RO 318220, RO 320432, GO 6976, Isis 3521, LY333531/LY379196, PD184352, QAN697, 4-Methyl-3-[[4-(3-pyridinyl)-2-pyrimidinyl]amino]-N-[5-(4-methyl-1H-imidazol-1-yl)-3-(trifluoromethyl)phenyl] benzamide, Tyrphostin A23/RG-50810, AG 99, Tyrphostin AG 213, Tyrphostin AG 1748, Tyrphostin AG 490, Tyrphostin B44, Tyrphostin B44 (+) enantiomer, Tyrphostin AG 555, AG 494, Tyrphostin AG 556, AG957, adaphostin (4-{[(2,5-dihydroxyphenyl)methyl]amino}-benzoic acid adamantyl ester, NSC 680410, adaphostin, CP 358774, ZD 1839, ZM105180, e.g., trastuzumab (HERCEPTIN®), cetuximab, Iressa, OSI-774, CI-1033, EKB-569, GW-2016, E1.1, E2.4, E2.5, E6.2, E6.4, E2.11, E6.3 or E7.6.3, erlotinib and gefitinib.

The present invention pertains to a pharmaceutical composition comprising a quantity which is jointly therapeutically effective against a proliferative disease of a combination as mentioned above.

The present invention pertains to the use of a combination as mentioned above for the preparation of a medicament for the treatment of a proliferative disease.

The present invention relates to a commercial package comprising a combination as mentioned above together with instructions.

### EXAMPLES

Example 1: Determination of synergistic anti-proliferative activities through combination of 4-[3,5-bis(2-hydroxyphenyl)-[1,2,4]triazol-1-yl]benzoic acid (herein after Compound I) with

### anti-neoplastic agents.

Compound I was tested for synergistic antiproliferative activity in combination with 5Fluorouracil, Mitomycin C, Cisplatin, Tumodex and Leucovorine against the human liver tumour cells HEP3B217 and SKHEP1 *in vitro.* The synergistic antiproliferative activity for Compound I with both combination partners with a series of standard chemotherapeutics was determined. The results for the two different tumour cell lines tested were quite different. Compound I antiproliferative activity was synergistic with all of the tested chemotherapeutics in HEP3B217 when dosed with concentrations lower than 12.0 µM. However, the level of synergism calculated by the G-Value was different with different combination partners and most pronounced with cisplatin. Compound I antiproliferative activity was much less synergistic in SKHEP1 cells; a synergistic mode of action could only be demonstrated for cisplatin and Tumodex. However, for both compounds the synergism of combination effects was demonstrated at concentrations lower than 1.5 µM or higher than µM. No synergism of action with these two compounds was found at concentrations higher than 12.5 µM with Tumodex or 25.0 µM with cisplatin.

### 3 Materials and Methods

### 3.1 Materials

Both the HEP3B217 and SKHEP1 cell lines were obtained from the American Type Culture Collection (ATCC), Rockville, MD, USA. RPMI 1640 cell culture medium and FCS were supplied by Invitrogen (Mt. Waverley, VIC, Australia). CellTitreBlue reagent was obtained from Promega (Annandale, NSW, Australia). Cell culture materials were of standard quality and obtained from commercial sources. Fine chemicals and reagents were of the highest purity available and purchased from local suppliers. Compound I was provided by the sponsor and details pertaining to the original source, identification and individual properties, as far as disclosed, are retained in the raw data file. 5Fluorouracil (Genentech, South San Francisco, CA, USA), Mitomycin C, Cisplatin, Leucovorine (BristolMyers Squibb, New York, NY, USA), and Tumodex (AstraZeneca Pharmaceuticals, Wilmington, DE, USA() were diluted in cell culture medium.

### 3.2 Test Compound Preparation

Compound I was dissolved in DMSO to produce a stock solution (1 mg/mL). This stock solution was thoroughly vortex mixed and diluted in cell culture medium to produce the desired starting concentration for the dilution series (1.3 µM) when added to the test well (50:50, v/v). 5-Fluorouracil, Mitomycin C, Cisplatin, Tumodex and Leucovorine were diluted in cell culture medium to the required starting concentrations for the dilutions series (see Table 1).

**Table 1: Starting concentrations of the test articles used for the combination studies**

| **Compound** | **ICL670** | **5FU** | **Leukoverine** | **Cisplatin** | **Mitomycin-C** | **Tomudex** |
|---|---|---|---|---|---|---|
| **HEP3B 2.1-7** | | | | | | |
| highest concentration [uM] | 50.00 | 20.00 | 50.00 | 20.00 | 30.00 | 50.00 |
| | | | | | | |
| **SK-HEP-1 (P4)** | | | | | | |
| highest conceratration [uM] | 50.00 | 1.00 | 50.00 | 10.00 | 1.50 | 1.50 |

### 3.3 Compound Administration, Schedule and Regimen

Both cell lines were cultured in RPMI 1640 cell culture medium containing 10 % FCS. The cell culture medium was supplemented with penicillin/streptomycin (50 IU/mL, 50 µg/mL final concentration). Both cell lines were seeded with 800 cells per well in 50 µL medium per well in 96well microtiter plates. 50 µL of cell culture medium containing the test compound(s) was added to each well twenty four hours after seeding. The test compounds were added as a single compound or as one of the required ratios of concentrations (Figure 1 details the dilution scheme (chequer board approach) for single compound or combinations). The final DMSO concentration in the incubation mixture was less than 0.05% (v/v) for the highest concentration test article and respectively lower for the lower concentration test articles. Proliferation of cells was assessed using the fluorimetric Alamar Blue (CellTiter Blue) test.

### 3.4 Data Acquisition

The Alamar Blue assay was used to determine the antiproliferative activity of the pools containing the test compounds. Untreated cells were used as growth controls. Data were assessed in duplicates using two identical setups in 96well microtiter plates. Each plate was analysed separately and isobolograms were calculated to determine synergistic activities (Figures 2 and 3). Furthermore, the gvalue was calculated using the following equation: g = Ac/Ae + Bc/Be, where A+B and A or B alone are at equally effective concentrations (e.g. IC₅₀), where Ac is the concentration of A in combination with B and Bc is the concentration of B in combination with A, Ae is the concentration of A alone and Be is the concentration of B alone. The gvalue represents the level of interaction of two combination partners. A gvalue < 1 indicates synergism, a gvalue >1 indicates antagonism and a gvalue = 1 indicates additive effects. The borders between these categories are indistinct and should be understood as a trend rather than as definitive decision points. However, the lower a gvalue the higher the synergistic effect is.

### 3.5 Alamar Blue Assay

The Alamar Bluebased CellTiterBlue assay indicates cell viability by measuring the ability of cells in culture to reduce resazurin to resorufin, whereby the intensity of the fluorescence signal is directly proportional to the number of live cells and hence an indirect indicator of cell proliferation.

Based on preliminary experiments, cultures of both cell lines were seeded at 0.8x10³ cells/well in 96well microtiter plates . After 24 hrs the cells were incubated for 72 hours with the single compound or combination of two test compounds. Following addition of CellTiterBlue Reagent (20 µL/well) and brief mixing, cells were incubated for another 4 hours before fluorescence was measured (Ex/Em of 560/590 nm) using a Spectramax M2 microplate reader (Molecular Devices, Surrey Hills, VIC, Australia).

### 4. Results

The anti-proliferative activity of Compound I was tested in combination with standard chemotherapeutics such as 5Fluorouracil, Mitomycin C, Cisplatin, Tumodex and Leucovorine. Compound I was tested at 9 concentrations starting with 50 µM followed by consecutive dilutions of 1:2. All other chemotherapeutics were tested at 8 concentrations using the starting (highest) concentrations shown in Table 1 and proceeding with 7 consecutive dilutions of 1:2. To assess the synergistic mode of action of Compound I in combination with standard chemotherapeutics the gvalue (refer to method section for equation) was calculated. If g < 1 the interaction can be considered as being synergistic. Gvalues equal to 1 indicate additive effects. If the gvalue is larger than 1 the interaction of the combined drugs is antagonistic (Tables 2 and 3, Figures 1 and 2). The gvalues were plotted in Isobolograms (data points below the isobolic line represent combined concentration of both drugs acting synergistically; data points above the isobolic line represent combined concentrations of both drugs acting antagonistically). However, these thresholds are not an absolute number and have rather to be interpreted in a dynamic way. To assess the optimal concentration of the combination partner, a Surface Response Plot was drawn. Each study was repeated once.

### Antiproliferative efficacy in SKHEP1 cells

Clear synergism of antiproliferative activity (g < 1) was observed only for combinations of Compound I with Cisplatin or Tumodex (Table 2). Interestingly for both combination partners two ranges of concentrations for ICL670 appear to produce synergism of antiproliferative action (between 25 µM and 3.25 µM and between 0.78 µM and 0.20 µM). Furthermore, a slight trend towards synergistic antiproliferative activity at concentrations of Compound I lower than 3 µM was demonstrated for the combination of Compound I with Mitomycin C (Table 2). The evaluation of the Surface Response Plot reveals that the concentration of Cisplatin or Tumodex combined with Compound I does play a minor role for synergistic action, whereas only lower concentrations of Compound I lead to synergistic action (Figure 1, Panels B and E).

**Table 2: Synergistic anti-proliferative effects of Compound I (ICL670 within the table) in combination with 5Fluorouracil, Mitomycin C, Cisplatin, Tumodex or Leucovorine in human SKHEP1 liver cancer cells.**

| **Combination with 5FU** | | | | | | | | | | | |
|---|---|---|---|---|---|---|---|---|---|---|---|
| | **Concentration ICL670 [uM]** | | **50.00** | **25.00** | **12.50** | **6.25** | **3.13** | **1.56** | **0.78** | **0.39** | **0.20** |
| **Study 1** | SK-HEP-1 | g-value | 19.87 | 12.21 | 8.17 | 6.77 | 6.22 | 2.18 | 4.09 | 2.04 | 2.13 |
| **Study 2** | SK-HEP-1 | g-value | 13.20 | 9.48 | 10.58 | 5.66 | 7.62 | 5.77 | 1.47 | 3.87 | 1.96 |

| | | | | | | | | | | | |
|---|---|---|---|---|---|---|---|---|---|---|---|
| **Combination with Cisplatin** | | | | | | | | | | | |
| | **Concentration ICL670 [uM]** | | **50.00** | **25.00** | **12.50** | **6.25** | **3.13** | **1.56** | **0.78** | **0.39** | **0.20** |
| **Study 1** | SK-HEP-1 | g-value | 1.78 | 0.34 | 0.47 | 0 | 1.52 | 1.28 | 0.80 | 0.95 | 1.21 |
| **Study 2** | SK-HEP-1 | g-value | 2.40 | 1.18 | 0.64 | 0.78 | 1.46 | 1 26 | 0.99 | 0.90 | 1.22 |

| | | | | | | | | | | | |
|---|---|---|---|---|---|---|---|---|---|---|---|
| **Combination with Leucoverin** | | | | | | | | | | | |
| | **Concentration ICL670 [uM]** | | **50.00** | **25.00** | **12.50** | **6.25** | **3.13** | **1.56** | **0.78** | **0.39** | **0.20** |
| **Study** 1 | SK-HEP-1 | g-value | 5427 | 12 | 112 | 3 | 5.E+0.7 | 21 | 543 | 764 | 492 |
| **Study 2** | SK-HET-1 | g-value | 1316.46 | 2542.07 | 11.15 | 40.52 | 1370.27 | 58.30 | 1.00 | 35.34 | 182.93 |
| | | | | | | | | | | | |

| **Combination with Mitomycin C** | | | | | | | | | | | |
|---|---|---|---|---|---|---|---|---|---|---|---|
| | **Concentration [CL670 [uM]** | | **50.00** | **25.00** | **12.50** | **6.25** | **3.13** | **1.56** | **0.78** | **0.39** | **0.20** |
| **Study1** | SK-HEF-1 | g-value | 34.71 | 11.81 | 32.51 | 3.23 | 3.88E+0.7 | 0.74 | 1.00 | 1.05 | 0.68 |
| **Study 2** | SK-HEP-1 | g-value | 24.82 | 31.18 | 6.21 | 34.56 | 159.97 | 0.92 | 2.02 | 2.18 | 1.28 |

| | | | | | | | | | | | |
|---|---|---|---|---|---|---|---|---|---|---|---|
| **Combination with Tumodex** | | | | | | | | | | | |
| | **Concentration ICL670 [uM]** | | **50.00** | **25.00** | **12.50** | **6.25** | **3.13** | **1.56** | **0.78** | **0.39** | **0.20** |
| **Study 1** | SK-HEP-1 | g-value | 3.54 | 1.38 | 0.84 | 4.53 | 2.46 | 2.27 | 1.55 | 0.92 | 0.98 |
| **Study 2** | SK-HEP-1 | g-value | 2.18 | 1.12 | 0.57 | 0.88 | 1197.98 | 1.19 | 0.68 | 0.84 | 0.68 |

### AntiProliferative efficacy in HEP3B217 cells

Compared to the action in SKHEP1 cells, Compound I did induce synergism of antiproliferative activity when combined with all the tested standard chemotherapeutics. The repeat study for the combination with Leoucoverin and Mitomycin C failed for reasons unknown (Table 3, rows labelled in grey colour). Therefore all conclusions for the combination of Compound I with these two drugs should be interpreted with caution. The strongest induction of synergism was observed when Compound I was combined with cisplatin; concentrations of Compound I lower than 12.5 µM combined with cisplatin over a broad range of concentrations produced synergistic mode of action (Table 3 and Figure 2, panel B: Isobologram and gvalue Plot). Furthermore, lower concentrations of Compound I combined with higher concentrations of cisplatin produced greater synergistic effects than Compound I combined with lower concentrations of cisplatin (Figure 2, panel B: Surface Response Plot). The combination of Compound I with Mitomycin C at concentrations between 6.25 µM and 0.781 µM (Tabel 3 and Figure 2, Panel D) also resulted in synergistic mode of action. However, compared to cisplatin the concentration of Mitomycin C had less influence on the level of synergistic interaction. As the gvalues are close to 1, the combination of Compound I with 5Fluoruracil resulted in much less pronounced synergism. Tumodex induced strong synergism at low concentrations of Compound I (<1.56 µM) in the first study. However, this could not be confirmed unequivocally in the second study (Table 3).

**Table 3: Synergistic antiproliferative effects of Compound I (ICL670 within the table) in combination with 5Fluorouracil, Mitomycin C, Cisplatin, Tumodex or Leucovorine in human HEP3B217 liver cancer cells.**

| **Combination with SFU** | | | | | | | | | | | |
|---|---|---|---|---|---|---|---|---|---|---|---|
| | **Concentration ICL670 [uM]** | | **50.000** | **25.000** | **12.500** | **6.250** | **3.125** | **1.663** | **0.781** | **0.391** | **0.20** |
| **Study 1** | HEP3B21.7 | g-value | 4.69 | 2.32 | 1.48 | 0.96 | 0.38 | 0.99 | 1.39 | 1.00 | 1.50 |
| **Study 2** | HEP3B21.7 | g-value | 4.65 | 2.67 | 1.22 | 0.74 | 1.25 | 0.91 | 1.22 | 1.35 | 0.84 |
| | | | | | | | | | | | |
| **Combination with Cisplatin** | | | | | | | | | | | |
| | **Concentration ICL670 [uM]** | | **50.000** | **25.000** | **12.500** | **6.250** | **3.125** | **1.663** | **0.781** | **0.391** | **0.20** |
| **Study 1** | HEP3B21.7 | g-value | 4.88 | 2.51 | 1.34 | 0.66 | 0.57 | 0.50 | 0.66 | 0.98 | 0.92 |
| **Study 2** | HEP3B21.7 | g-value | 3.30 | 1.68 | 0.96 | 0.64 | 0.54 | 0.56 | 0.81 | 0.96 | 0.99 |
| | | | | | | | | | | | |
| **Combination with Leucoverin** | | | | | | | | | | | |
| | **Concentration [uM]** | **ICL670** | **50.000** | **25.000** | **12.500** | **6.250** | **3.125** | **1.563** | **0.781** | **0.391** | **0.20** |
| **Study 1** | HEP3B21.7 | g-value | 4.58 | 2.32 | 1.47 | 0.98 | 0.82 | 1.19 | 3.99 | 9.62 | 0.44 |
| **Study 2** | HEP3B21.7 | g-value | 424 | 256 | 1198 | 1581 | 2587 | 2384 | 54118 | 1577291 | 10.00 |
| | | | | | | | | | | | |
| **Combination with Mitomycin C** | | | | | | | | | | | |
| | **Concentration ICL670 [uM]** | | **50.000** | **25.000** | **12.500** | **6.260** | **3.125** | **1.563** | **0.781** | **0.391** | **0.20** |
| **Study 1** | HEP3B21.7 | g-value | 4.61 | 2.50 | 1.25 | 0.73 | 0.82 | 0.18 | 0.57 | 185.04 | 8.37 |
| **Study 2** | HEP3B21.7 | g-value | 5.00 | 82.61 | 17.05 | 10.95 | 2087 | 2384 | 316.78 | 1519.50 | 2324.01 |
| | | | | | | | | | | | |
| **Combination with Tumodex** | | | | | | | | | | | |
| | **Concentration ICL670 [uM]** | | **60.00.0** | **26.000** | **12.500** | **6.250** | **3.125** | **1.563** | **0.781** | **0.391** | **0.20** |
| **Study 1** | HEP3B21.7 | g-value | 17.42 | 588.11 | 33.13 | 8.67 | 9.16 | 0.88 | 0.17 | 0.13 | 0.20 |
| **Study 2** | HEP3B20.7 | g-value | 406.06 | 563.15 | 538.15 | 155.27 | 129.30 | 70.13 | 0.18 | 180.49 | 0.03 |

For Compound I in combination with the standard combination therapeutics 5Fluorouracil, Mitomycin C, Cisplatin, Tumodex or Leucovorine, the potential for synergism of antiproliferative activity was determined *in vitro* in human SKHEP1 and HEP3B217 liver cancer cells using *vivo*Pharm's optimised chequerboard assay. In both cell lines cisplatin induced the highest level of synergism, followed by Tumodex in SKHEP1 cells and 5Fluorouracil in HEP3B217 cells. Compound I can inhibit proliferation in human liver cancer cell lines synergistically. The observed level of synergism was much higher in HEP3B217 cells than in SKHEP1 cells, and was most pronounced in combination with cisplatin.

## Claims

1. A combination which comprises (a) a substituted 3,5-diphenyl-1,2,4-triazole and (b) a platinum compound and, wherein the active ingredients are present in each case in free form or in the form of a pharmaceutically acceptable salt, and optionally at least one pharmaceutically acceptable carrier.

2. The combination according to claim 1 for simultaneous, separate or sequential use in the treatment of the human body.

3. The combination according to claim 2 wherein (b) the platinum compound is selected from the group consisting of carboplatin, cisplatin, and oxaliplatin.

4. The combination according to claim 3 wherein (b) is cisplatin.

5. The combination according to any one of claims 1 to 4 wherein (a) is 4-[3,5-bis(2-hydroxyphenyl)-[1,2,4]triazol-1-yl]benzoic acid.

6. A pharmaceutical composition comprising a quantity which is jointly therapeutically effective against a proliferative disease of a combination according to any one of claims 1 to 5 and at least one pharmaceutically acceptable carrier.

7. Use of a combination according to any one of claims 1 to 5 for the preparation of a medicament for the treatment of a proliferative disease.

8. A commercial package comprising a combination according to anyone of claims 1 to 5 together with instructions.
